(19) Europäisches Patentamt
European Patent Office
Office européen des brevets

(11) EP 1 497 661 B1

# (12) EUROPEAN PATENT SPECIFICATION

(45) Date of publication and mention of the grant of the patent:
**25.11.2009 Bulletin 2009/48**

(21) Application number: **03720520.0**

(22) Date of filing: **24.04.2003**

(51) Int Cl.:
***G01N 33/68*** *(2006.01)* ***A01K 67/027*** *(2006.01)*
***C12Q 1/68*** *(2006.01)*

(86) International application number:
**PCT/EP2003/004257**

(87) International publication number:
**WO 2003/091734 (06.11.2003 Gazette 2003/45)**

(54) **DIAGNOSTIC USE OF ENSADIN-0477 GENE AND PROTEIN FOR NEURODEGENERATIVE DISEASES**

DIAGNOSTISCHE VERWENDUNG VON "ENSADIN-0477" FÜR NEURODEGENERATIVE ERKRANKUNGEN

UTILISATION A DES FINS DIAGNOSTIQUES DU GENE ET DE LA PROTEINE ENSADINE-0477 POUR LE TRAITEMENT DE MALADIES NEURODEGENERATIVES

(84) Designated Contracting States:
**AT BE BG CH CY CZ DE DK EE ES FI FR GB GR HU IE IT LI LU MC NL PT RO SE SI SK TR**

(30) Priority: **24.04.2002 EP 02009139**
**24.04.2002 US 374816 P**

(43) Date of publication of application:
**19.01.2005 Bulletin 2005/03**

(73) Proprietor: **EVOTEC Neurosciences GmbH**
**22525 Hamburg (DE)**

(72) Inventors:
- **VON DER KAMMER, Heinz**
  **22607 Hamburg (DE)**
- **POHLNER, Johannes**
  **Hamburg 22175 (DE)**
- **HANES, Jozef**
  **84107 Bratislava (SK)**

(74) Representative: **Meyers, Hans-Wilhelm et al**
**Patentanwälte**
**von Kreisler Selting Werner**
**Postfach 10 22 41**
**50462 Köln (DE)**

(56) References cited:
**EP-A- 1 293 569** **WO-A-00/70099**
**WO-A-02/16636** **WO-A-02/22867**

- **DATABASE EMBL [Online] 31 October 2001 (2001-10-31) " Homo sapiens cDNA FLJ31673 fis, clone NT2RI2005061" retrieved from EBI Database accession no. AK056235 XP002242520**
- **DAVIES P ET AL: "CONSENSUS REPORT OF THE WORKING GROUP ON: MOLECULAR AND BIOCHEMICAL MARKERS OF ALZHEIMER'S DISEASE" NEUROBIOLOGY OF AGING, TARRYTOWN, NY, US, vol. 19, no. 2, 1998, pages 109-116, XP001148347 ISSN: 0197-4580**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention relates to methods of diagnosing, prognosticating, and monitoring the progression of neurodegenerative diseases in a subject. The invention also discloses the use of kits, and recombinant mouse models.

Neurodegenerative diseases, in particular Alzheimer's disease (AD), have a strongly debilitating impact on a patient's life. Furthermore, these diseases constitute an enormous health, social, and economic burden. AD is the most common neurodegenerative disease, accounting for about 70% of all dementia cases, and it is probably the most devastating age-related neurodegenerative condition affecting about 10% of the population over 65 years of age and up to 45% over age 85 (for a recent review see Vickers et al., Progress in Neurobiology 2000, 60: 139-165). Presently, this amounts to an estimated 12 million cases in the US, Europe, and Japan. This situation will inevitably worsen with the demographic increase in the number of old people ("aging of the baby boomers") in developed countries. The neuropathological hallmarks that occur in the brains of individuals with AD are senile plaques, composed of amyloid-β protein, and profound cytoskeletal changes coinciding with the appearance of abnormal filamentous structures and the formation of neurofibrillary tangles.

The amyloid-β (Aβ) protein evolves from the cleavage of the amyloid precursor protein (APP) by different kinds of proteases. The cleavage by the β/γ-secretase leads to the formation of Aβ peptides of different lengths, typically a short more soluble and slow aggregating peptide consisting of 40 amino acids and a longer 42 amino acid peptide, which rapidly aggregates outside the cells, forming the characteristic amyloid plaques (Selkoe, Physiological Rev 2001, 81: 741-66; Greenfield et al., Frontiers Bioscience 2000, 5: D72-83). Two types of plaques, diffuse plaques and neuritic plaques, can be detected in the brain of AD patients, the latter ones being the classical, most prevalent type. They are primarily found in the cerebral cortex and hippocampus. The neuritic plaques have a diameter of 50μm to 200μm and are composed of insoluble fibrillar amyloids, fragments of dead neurons, of microglia and astrocytes, and other components such as neurotransmitters, apolipoprotein E, glycosaminoglycans, α1-antichymotrypsin and others. The generation of toxic Ap deposits in the brain starts very early in the course of AD, and it is discussed to be a key player for the subsequent destructive processes leading to AD pathology. The other pathological hallmarks of AD are neurofibrillary tangles (NFTs) and abnormal neurites, described as neuropil threads (Braak and Braak, Acta Neuropathol 1991, 82: 239-259). NFTs emerge inside neurons and consist of chemically altered tau, which forms paired helical filaments twisted around each other. Along the formation of NFTs, a loss of neurons can be observed. It is discussed that said neuron loss may be due to a damaged microtubule-associated transport system (Johnson and Jenkins, J Alzheimers Dis 1996, 1: 38-58; Johnson and Hartigan, J Alzheimers Dis 1999, 1: 329-351). The appearance of neurofibrillary tangles and their increasing number correlates well with the clinical severity of AD (Schmitt et al., Neurology 2000, 55: 370-376).
AD is a progressive disease that is associated with early deficits in memory formation and ultimately leads to the complete erosion of higher cognitive function. The cognitive disturbances include among other things memory impairment, aphasia, agnosia and the loss of executive functioning. A characteristic feature of the pathogenesis of AD is the selective vulnerability of particular brain regions and subpopulations of nerve cells to the degenerative process. Specifically, the temporal lobe region and the hippocampus are affected early and more severely during the progression of the disease. On the other hand, neurons within the frontal cortex, occipital cortex, and the cerebellum remain largely intact and are protected from neurodegeneration (Terry et al., Annals of Neurology 1981, 10: 184-92). the age of onset of AD may vary within a range of 50.years, with early-onset AD occurring in people younger than 65 years of age, and late-onset of AD occurring in those older than 65 years. About 10% of all AD cases suffer from early-onset AD, with only 1-2% being familial, inherited cases.

Currently, there is no cure for AD, nor is there an effective treatment to halt the progression of AD or even to diagnose AD ante-mortem with high probability. Several risk factors have been identified that predispose an individual to develop AD, among them most prominently the epsilon 4 allele of the three different existing alleles (epsilon 2, 3, and 4) of the apolipoprotein E gene (ApoE) (Strittmatter et al., Proc Natl Acad Sci USA 1993, 90: 1977-81; Roses, Ann NY Acad Sci 1998, 855: 738-43). The polymorphic plasmaprotein ApoE plays a role in the intercellular cholesterol and phospholipid transport by binding low-density lipoprotein receptors, and it seems to play a role in neurite growth and regeneration. Efforts to detect further susceptibility genes and disease-linked polymorphisms, lead to the assumption that specific regions and genes on human chromosomes 10 and 12 may be associated with late-onset AD (Myers et al., Science 2000, 290: 2304-5; Bertram et al., Science 2000, 290: 2303; Scott et al., Am J Hum Genet 2000, 66: 922-32).

Although there are rare examples of early-onset AD which have been attributed to genetic defects in the genes for amyloid precursor protein (APP) on chromosome 21, presenilin-1 on chromosome 14, and presenilin-2 on chromosome 1, the prevalent form of late-onset sporadic AD is of hitherto unknown etiologic origin. The mutations found to date account for only half of the familial AD cases, which is less than 2% of all AD patients. The late onset and complex pathogenesis of neurodegenerative disorders pose a formidable challenge to the development of therapeutic and diagnostic agents. It is crucial to expand the pool of potential drug targets and diagnostic markers. It is therefore an object of the present invention to provide insight into the pathogenesis of neurological diseases and to provide methods, materials, agents, compositions, and animal models which are suited inter alia for the diagnosis and development of a treatment of these diseases. This object has been solved by the features of the independent claims. The subclaims define preferred embodiments of the present invention.

In the present invention, the differential expression of a gene coding for Ensadin-0477 is detected in human Alzheimer's disease brain samples by using an unbiased and sensitive differential display technology. The Ensadin-0477 gene is located on the human X-chromosome at position Xq22.3-23. The gene harbors a predicted open reading frame coding for a protein of 937 amino acids in length, (SEQ ID NO. 1), with an approximate molecular weight of about 106 kDa and with no obvious homologies to other known proteins. The protein contains an ATPase-like ATP-binding region (SEQ ID No. 1, amino acids 45 to 143) which shares striking similarities to, and thus, may belong to the family representing the structurally related ATPase domains of histidine kinase, DNA gyrase B and HSP90 (SwissEntry: Q8TE76, Pfam PF02518, HATPase_c). The human cDNA of Ensadin-0477 is identical to a deduced human cDNA clone in the GenBank database (accession number dJ75H8.2) and is part of a large human genomic sequence (accession number AL158821). However, the predicted open reading frame of the human Ensadin-0477 cDNA differs from the predicted open reading frame of cDNA sequence dJ75H8.2 in 34 additional amino acids at its N-terminus. The alignment of human Ensadin-0477 cDNA, (SEQ ID NO. 2), with sequences contained in the GenBank database produces a homologous human expressed sequence tag (EST) nucleotide sequence (au118268) and partial cDNA fragments (ak056235, ak021627). They all differ from the nucleotide sequence of Ensadin-0477 cDNA in having one or three nucleic acids exchanged. The following nucleotides are altered in cDNA clone ak056235 (positions according to SEQ ID NO. 2): 1007 A/T (Val244Val); 1194 T/C (Tyr307His); 1693 C/T (Thr473Ile). The cDNA clone ak021627 and the EST sequence au118268 are altered in the same way as clone ak056235, at nucleotide 1693 of SEQ ID NO. 2. These nucleotide alterations may reflect the existence of single nucleotide polymorphisms, or variants, or isoforms of Ensadin-0477. The present invention discloses a dysregulation of Ensadin-0477 gene expression on the transcriptional level in the inferior temporal lobe relative to frontal lobe and in the hippocampus relative to frontal lobe of brain samples taken from AD patients. No such dysregulation is observed in samples from age-matched healthy controls. To date, no experiments have been described that demonstrate a relationship between the dysregulation of Ensadin-0477 gene expression and the pathology of neurodegenerative diseases, in particular AD. Such a link, as disclosed in the present invention, offers new ways, inter alia, for the diagnosis and treatment of said diseases.

The singular forms "a", "an", and "the" as used herein and in the claims include plural reference unless the context dictates otherwise. For example, "a cell" means as well a plurality of cells, and so forth. The term "and/or" as used in the present specification and in the claims implies that the phrases before and after this term are to be considered either as alternatives or in combination. For instance, the wording "determination of a level and/or an activity" means that either only a level, or only an activity, or both a level and an activity are determined. The term "level" as used herein is meant to comprise a gage of, or a measure of the amount of, or a concentration of a transcription product, for instance an mRNA, or a translation product, for instance a protein or polypeptide. The term "activity" as used herein shall be understood as a measure for the ability of a transcription product or a translation product to produce a biological effect or a measure for a level of biologically active molecules. The term "activity" also refers to enzymatic activity. The terms "level" and/or "activity" as used herein further refer to gene expression levels or gene activity. Gene expression can be defined as the utilization of the information contained in a gene by transcription and translation leading to the production of a gene product. "Dysregulation" shall mean an upregulation or downregulation of gene expression. A gene product comprises either RNA or protein and is the result of expression of a gene. The amount of a gene product can be used to measure how active a gene is. The term "gene" as used in the present specification and in the claims comprises both coding regions (exons) as well as non-coding regions (e.g. non-coding regulatory elements such as promoters or enhancers, introns, leader and trailer sequences). The term "ORF" is an acronym for "open reading frame" and refers to a nucleic acid sequence that does not possess a stop codon in at least one reading frame and therefore can potentially be translated into a sequence of amino acids. "Regulatory elements" shall comprise inducible and non-inducible promoters, enhancers, operators, and other elements that drive and regulate gene expression. The term "fragment" as used herein is meant to comprise e.g. an alternatively spliced, or truncated, or otherwise cleaved transcription product or translation product. The term "derivative" as used herein refers to a mutant, or an RNA-edited, or a chemically modified, or otherwise altered transcription product, or to a mutant, or chemically modified, or otherwise altered translation product. For instance, a "derivative" may be generated by processes such as altered phosphorylation, or glycosylation, or acetylation, or lipidation, or by altered signal peptide cleavage or other types of maturation cleavage. These processes may occur post-translationally. The term "modulator" as used in the present invention and in the claims refers to a molecule capable of changing or altering the level and/or the activity of a gene, or a transcription product of a gene, or a translation product of a gene. Preferably, a "modulator" is capable of changing or altering the biological activity of a transcription product or a translation product of a gene. Said modulation, for instance, may be an increase or a decrease in enzyme activity, a change in binding characteristics, or any other change or alteration in the biological, functional, or immunological properties of said translation product of a gene. The terms "agent", "reagent", or "compound" refer to any substance, chemical, composition or extract that have a positive or negative biological effect on a cell, tissue, body fluid, or within the context of any biological system, or any assay system examined. They can be agonists, antagonists, partial agonists or inverse agonists of a target. Such agents, reagents, or compounds may be nucleic acids, natural or synthetic peptides or protein complexes, or fusion proteins. They may also be antibodies, organic or anorganic molecules or compositions, small molecules, drugs and any combinations of any of said agents above. They may be used for testing, for diagnostic or for therapeutic purposes. The terms "oligonucleotide primer" or "primer" refer to short nucleic acid sequences which can anneal to a given target polynucleotide by hybridization of the complementary base pairs and can be extended by a polymerase. They may be chosen to be specific to a particular sequence or they may be randomly selected, e.g. they will prime all possible sequences in a mix. The length of primers used herein may vary from 10 nucleotides to 80 nucleotides. "Probes" are short nucleic acid sequences of the nucleic acid sequences described and disclosed herein or sequences complementary therewith. They may comprise full length sequences, or fragments, derivatives, isoforms, or variants of a given sequence. The identification of hybridization complexes between a "probe" and an assayed sample allows the detection of the presence of other similar sequences within that sample. As used herein, "homolog or homology" is a term used in the art to describe the relatedness of a nucleotide or peptide sequence to another nucleotide or peptide sequence, which is determined by the degree of identity and/or similarity between said sequences compared.
The term "variant" as used herein refers to any polypeptide or protein, in reference to polypeptides and proteins disclosed in the present invention, in which one or more amino acids are added and/or substituted and/or deleted and/or inserted at the N-terminus, and/or the C-terminus, and/or within the native amino acid sequences of the native polypeptides or proteins of the present invention. Furthermore, the term "variant" shall include any shorter or longer version of a polypeptide or protein. "Variants" shall also comprise a sequence that has at least about 80% sequence identity, more preferably at least about 90% sequence identity, and most preferably at least about 95% sequence identity with the amino acid sequences of SEQ ID NO.1. "Variants" of a protein molecule shown in SEQ ID NO. 1 include, for example, proteins with conservative amino acid substitutions in highly conservative regions. "Proteins and polypeptides" of the present invention, correspond to the protein comprising SEQ ID NO. 1. They can include proteins and polypeptides which can be isolated from nature or be produced by recombinant and/or synthetic means. Native proteins or polypeptides refer to naturally-occurring truncated or secreted forms, naturally occurring variant forms (e.g. splice-variants) and naturally occurring allelic variants. The term "isolated" as used herein is considered to refer to molecules that are removed from their natural environment, i.e. isolated from a cell or from a living organism in which they normally occur, and that are separated or essentially purified from the coexisting components with which they are found to be associated in nature. This notion further means that the sequences encoding such molecules can be linked by the hand of man to polynucleotides, to which they are not linked in their natural state, and that such molecules can be produced by recombinant and/or synthetic means. Even if for said purposes those sequences may be introduced into living or non-living organisms by methods known to those skilled in the art, and even if those sequences are still present in said organisms, they are still considered to be isolated. In the present invention, the terms "risk", "susceptibility", and "predisposition" are tantamount and are used with respect to the probability of developing a neurodegenerative disease, preferably Alzheimer's disease.
The term 'AD' shall mean Alzheimer's disease. "AD-type neuropathology" as used herein refers to neuropathological, neurophysiological, histopathological and clinical hallmarks as described in the instant invention and as commonly known from state-of-the-art literature (see: Iqbal, Swaab, Winblad and Wisniewski, Alzheimer's Disease and Related Disorders (Etiology, Pathogenesis and Therapeutics), Wiley & Sons, New York, Weinheim, Toronto, 1999; Scinto and Daffner, Early Diagnosis of Alzheimer's Disease, Humana Press, Totowa, New Jersey, 2000; Mayeux and Christen, Epidemiology of Alzheimer's Disease: From Gene to Prevention, Springer Press, Berlin, Heidelberg, New York, 1999; Younkin, Tanzi and Christen, Presenilins and Alzheimer's Disease, Springer Press, Berlin, Heidelberg, New York, 1998).
Neurodegenerative diseases or disorders according to the present invention comprise Alzheimer's disease, Parkinson's disease, Huntington's disease, amyotrophic lateral sclerosis, Pick's disease, fronto-temporal dementia, progressive nuclear palsy, corticobasal degeneration, cerebro-vascular dementia, multiple system atrophy, argyrophilic grain dementia and other tauopathies, and mild-cognitive impairment. Further conditions involving neurodegenerative processes are, for instance, age-related macular degeneration, narcolepsy, motor neuron diseases, prion diseases, traumatic nerve injury and repair, and multiple sclerosis.

In one aspect, the invention features a method of diagnosing or prognosticating a neurodegenerative disease in a subject, or determining whether a subject is at increased risk of developing said disease. The method comprises: determining a level, or an activity, or both said level and said activity of (i) a transcription product of a gene having the nucleotide sequence of SEQ ID No. 2, and/or of (ii) a translation product of a gene having the nucleotide sequence of SEQ ID No. 2, in a sample from said subject and comparing said level, and/or said activity to a reference value representing a known disease or health status, thereby diagnosing or prognosticating said neurodegenerative disease in said subject, or determining whether said subject is at increased risk of developing said neurodegenerative disease.

The invention also relates to the construction and the use of primers and probes which are unique to the nucleic acid sequences as disclosed in the present invention. The oligonucleotide primers and/or probes can be labeled specifically with fluorescent, bioluminescent, magnetic, or radioactive substances. The invention further relates to the detection and the production of said nucleic acid sequences, or fragments and/or variants thereof, using said specific oligonucleotide primers in appropriate combinations. PCR-analysis, a method well known to those skilled in the art, can be performed with said primer combinations to amplify said gene specific nucleic acid sequences from a sample containing nucleic acids. Such sample may be derived either from healthy or diseased subjects. Whether an amplification results in a specific nucleic acid product or not, and whether a fragment of different length can be obtained or not, may be indicative for a neurodegenerative disease, in particular Alzheimer's disease. Thus, the invention provides nucleic acid sequences, oligonucleotide primers, and probes of at least 10 bases in length up to the entire coding and gene sequences, useful for the detection of gene mutations and single nucleotide polymorphisms in a given sample comprising nucleic acid sequences to be examined, which may be associated with neurodegenerative diseases, in particular Alzheimer's disease. This feature has utility for developing rapid DNA-based diagnostic tests, preferably also in the format of a kit.

In a further aspect, the invention features a method of monitoring the progression of a neurodegenerative disease in a subject. A level, or an activity, or both said level and said activity, of (i) a transcription product of a gene having the nucleotide sequence of SEQ ID No. 2, and/or of (ii) a translation product of a gene having the nucleotide sequence of SEQ ID No. 2 in a sample from said subject is determined. Said level and/or said activity is compared to a reference value representing a known disease or health status. Thereby the progression of said neurodegenerative disease in said subject is monitored.

In still a further aspect, the invention features a method of evaluating a treatment for a neurodegenerative disease, comprising determining a level, or an activity, or both said level and said activity of (i) a transcription product of a gene having the nucleotide sequence of SEQ ID No. 2, and/or of (ii) a translation product of a gene having the nucleotide sequence of SEQ ID No. 2 in a sample obtained from a subject being treated for said disease. Said level, or said activity, or both said level and said activity are compared to a reference value representing a known disease or health status, thereby evaluating the treatment for said neurodegenerative disease.

In a preferred embodiment of the herein claimed methods, use of the kits, recombinant mice, molecules, assays, and uses of the instant invention, said gene coding for Ensadin-0477 protein is the gene as shown in SEQ ID NO. 2. Likewise, said Ensadin-0477 protein is the protein as shown in SEQ ID NO. 1.

In a further preferred embodiment of the herein claimed methods, use of the kits, recombinant animals, molecules, assays, and uses of the instant invention, said neurodegenerative disease or disorder is Alzheimer's disease, and said subjects suffer from Alzheimer's disease.

The present invention discloses the detection and differential expression and regulation of a gene having the nucleotide sequence of SEQ ID No. 2 in specific brain regions of AD patients. Consequently, the Ensadin-0477 gene and its corresponding transcription and/or translation products may have a causative role in the regional selective neuronal degeneration typically observed in AD. Alternatively, Ensadin-0477 may confer a neuroprotective function to the remaining surviving nerve cells. Based on these disclosures, the present invention has utility for the diagnostic evaluation and prognosis as well as for the identification of a predisposition to a neurodegenerative disease, in particular AD. Furthermore, the present invention provides methods for the diagnostic monitoring of patients undergoing treatment for such a disease.

It is particularly preferred that said sample to be analyzed and determined is selected from the group comprising brain tissue, or other tissues, or body cells. The sample can also comprise cerebrospinal fluid or other body fluids including saliva, urine, blood, serum plasma, or mucus. Preferably, the methods of diagnosis, prognosis, monitoring the progression or evaluating a treatment for a neurodegenerative disease, according to the instant invention, can be practiced *ex corpore,* and such methods preferably relate to samples, for instance, body fluids or cells, removed, collected, or isolated from a subject or patient.

In further preferred embodiments, said reference value is that of a level, or an activity, or both said level and said activity of (i) a transcription product of a gene having the nucleotide sequence of SEQ ID No.2, and/or of (ii) a translation product of a gene having the nucleotide sequence of SEQ ID No.2 in a sample from a subject not suffering from said neurodegenerative disease.

In preferred embodiments, an alteration in the level and/or activity of a transcription product of a gene having the nucleotide sequence of SEQ ID No.2 and/or of a translation product of a gene having the nucleotide sequence of SEQ ID No.2 in a sample cell, or tissue, or body fluid from said subject relative to a reference value representing a known health status indicates a diagnosis, or prognosis, or increased risk of becoming diseased with AD.

In preferred embodiments, measurement of the level of transcription products of an Ensadin-0477 gene is performed in a sample from a subject using a quantitative PCR-analysis with primer combinations to amplify said gene specific sequences from cDNA obtained by reverse transcription of RNA extracted from a sample of a subject. A Northern blot with probes specific for said gene can also be applied. It might further be preferred to measure transcription products by means of chip-based microarray technologies. These techniques are known to those of ordinary skill in the art (see Sambrook and Russell, Molecular Cloning: A Laboratory Manual, Cold Spring Harbor Laboratory Press, Cold Spring Harbor, New York, 2001; Schena M., Microarray Biochip Technology, Eaton Publishing, Natick, MA, 2000). An example of an immunoassay is the detection and measurement of enzyme activity as disclosed and described in the patent application WO 02/14543.

Furthermore, a level and/or an activity of a translation product of an Ensadin-0477 gene having the nucleotide sequence of SEQ ID No.2 and/or a level of activity of said translation product can be detected using an immunoassay, an activity assay, and/or a binding assay. These assays can measure the amount of binding between said protein molecule and an anti-protein antibody by the use of enzymatic, chromodynamic, radioactive, magnetic, or luminescent labels which are attached to either the anti-protein antibody or a secondary antibody which binds the anti-protein antibody. In addition, other high affinity ligands may be used. Immunoassays which can be used include e.g. ELISAs, Western blots, and other techniques known to those of ordinary skill in the art (see Harlow and Lane, Using Antibodies: A Laboratory Manual, Cold Spring Harbor Laboratory Press, Cold Spring Harbor, New York, 1999 and Edwards R, Immunodiagnostics: A Practical Approach, Oxford University Press, Oxford; England, 1999). All these detection techniques may also be employed in the format of microarrays, protein-arrays, antibody microarrays, tissue microarrays, electronic biochip or protein-chip based technologies (see Schena M., *Microarray Biochip Technology,* Eaton Publishing, Natick, MA, 2000).

In a preferred embodiment, the level, or the activity, or both said level and said activity of (i) a transcription product of a gene having the nucleotide sequence of SEQ ID No.2, and/or of (ii) a translation product of a gene having the nucleotide sequence of SEQ ID No.2 in a series of samples taken from said subject over a period of time is compared, in order to monitor the progression of said disease. In further preferred embodiments, said subject receives a treatment prior to one or more of said sample gatherings. In yet another preferred embodiment, said level and/or activity is determined before and after said treatment of said subject.

In another aspect, the invention features the use of a kit for diagnosing or prognosticating neurodegenerative diseases, in particular AD, in a sample of a subject, or determining the propensity or predisposition of a subject to develop a neurodegenerative disease, in particular AD, said kit comprising:

at least one reagent which is selected from the group consisting of (i) reagents that selectively detect a transcription product of a gene having the nucleotide sequence of SEQ ID No.2, and (ii) reagents that selectively detect a translation product of a gene having the nucleotide sequence of SEQ ID No.2

said use being effected by:

- detecting a level, or an activity, or both said level and said activity, of said transcription product and/or said translation product of a gene having the nucleotide sequence of SEQ ID No.2, in a sample from said subject; and
- diagnosing or prognosticating a neurodegenerative disease, in particular AD, or determining the propensity or predisposition of said subject to develop such a disease,

wherein a varied level, or activity, or both said level and said activity, of said transcription product and/or said translation product compared to a reference value representing a known health status; or a level, or activity, or both said level and said activity, of said transcription product and/or said translation product similar or equal to a reference value representing a known disease status, indicates a diagnosis or prognosis of a neurodegenerative disease, in particular AD, or an increased propensity or predisposition of developing such a disease. The kit, according to the present invention is used for the identification of individuals that are at risk of developing a neurodegenerative disease, in particular AD. Consequently, the kit, according to the invention, may serve as a means for targeting identified individuals for early preventive measures or therapeutic intervention prior to disease onset, before irreversible damage in the course of the disease has been inflicted. Furthermore, in preferred embodiments, the kit is used for monitoring a progression of a neurodegenerative disease, in particular AD, in a subject, as well as monitoring success or failure of therapeutic treatment for such a disease of said subject.

In preferred embodiments, the subject for prevention,according to the present invention, can be a human, a mouse. The experimental mouse can be a mouse model for a neurodegenerative disorder, e.g. a transgenic mouse and/or a knock-out mouse with an AD-type neuropathology.

In a further aspect, the invention features a recombinant, mouse comprising a non-native gene sequence having the nucleotide sequence of SEQ ID No.2. The generation of said recombinant, non-human mouse comprises (i) providing a gene targeting construct containing said gene sequence and a selectable marker sequence, and (ii) introducing said targeting construct into a stem cell of a mouse, and (iii) introducing said mouse stem cell into a mouse embryo, and (iv) transplanting said embryo into a pseudopregnant mouse, and (v) allowing said embryo to develop to term, and (vi) identifying a genetically altered mouse whose genome comprises a modification of said gene sequence in both alleles, and (vii) breeding the genetically altered mouse of step (vi) to obtain a genetically altered mouse whose genome comprises a modification of said endogenous gene, wherein said gene is mis-expressed, or under-expressed, or over-expressed, and wherein said disruption or alteration results in said mouse exhibiting a predisposition to developing symptoms of neuropathology similar to a neurodegenerative disease, in particular AD. Strategies and techniques for the generation and construction of such a mouse are known to those of ordinary skill in the art (see e.g. Capecchi, Science 1989, 244: 1288-1292 and Hogan et al., 1994, Manipulating the Mouse Embryo: A Laboratory Manual, Cold Spring Harbor Laboratory Press, Cold Spring Harbor, New York and Jackson and Abbott, Mouse Genetics and Transgenics: A Practical Approach, Oxford University Press, Oxford, England, 1999). It is preferred to make use of such a recombinant mouse as an animal model for investigating neurodegenerative diseases, in particular Alzheimer's disease. Such a mouse may be useful for screening, testing and validating compounds, agents and modulators in the development of diagnostics for neurodegenerative diseases, in particular for Alzheimer's disease.

The present invention features a protein molecule shown in SEQ ID NO. 1.

The present invention further features a protein molecule shown in SEQ ID NO. 1, said protein molecule being a translation product of the gene having the nucleotide sequence of SEQ ID No.2.

The present invention features an antibody which is specifically immunoreactive with an immunogen, wherein said immunogen is a protein having the amino acid sequence of SEQ ID NO.1. The immunogen may comprise immunogenic or antigenic epitopes or portions of a translation product of said gene, wherein said immunogenic or antigenic portion of a translation product is a polypeptide, and wherein said polypeptide elicits an antibody response in an animal, and wherein said polypeptide is immunospecifically bound by said antibody. Methods for generating antibodies are well known in the art (see Harlow et al., Antibodies, A Laboratory Manual, Cold Spring Harbor Laboratory Press, Cold Spring Harbor, New York, 1988). The term "antibody", as employed in the present invention, encompasses all forms of antibodies known in the art, such as polyclonal, monoclonal, chimeric, recombinatorial, anti-idiotypic, humanized, or single chain antibodies, as well as fragments thereof (see Dubel and Breitling, Recombinant Antibodies, Wiley-Liss, New York, NY, 1999). Antibodies of the present invention are useful, for instance, in a variety of diagnostic and therapeutic methods, based on state-in-the-art techniques (see Harlow and Lane, Using Antibodies: A Laboratory Manual, Cold Spring Harbor Laboratory Press, Cold Spring Harbor, New York, 1999 and Edwards R., Immunodiagnostics: A Practical Approach, Oxford University Press, Oxford, England, 1999) such as enzyme-immuno assays (e.g. enzyme-linked immunosorbent assay, ELISA), radioimmuno assays, chemoluminescence-immuno assays, Western-blot, immunoprecipitation and antibody microarrays. These methods involve the detection of translation products of the Ensadin-0477 gene.

In a preferred embodiment of the present invention, said antibodies can be used for detecting the pathological state of a cell in a sample from a subject, comprising immunocytochemical staining of said cell with said antibody, wherein an altered degree of staining, or an altered staining pattern in said cell compared to a cell representing a known health status indicates a pathological state of said cell, wherein the pathological state relates to a neurodegenerative disease, in particular to AD. Immunocytochemical staining of a cell can be carried out by a number of different experimental methods well known in the art. It might be preferred, however, to apply an automated method for the detection of antibody binding, wherein the determination of the degree of staining of a cell, or the determination of the cellular or subcellular staining pattern of a cell, or the topological distribution of an antigen on the cell surface or among organelles and other subcellular structures within the cell, are carried out according to the method described in US patent 6150173.

Other features and advantages of the invention will be apparent from the following description of figures and examples which are illustrative only and not intended to limit the remainder of the disclosure in any way.

Figure 1 depicts the brain regions with selective vulnerability to neuronal loss and degeneration in AD. Primarily, neurons within the inferior temporal lobe, the entorhinal cortex, the hippocampus, and the amygdala are subject to degenerative processes in AD (Terry et al., Annals of Neurology 1981, 10:184-192). These brain regions are mostly involved in the processing of learning and memory functions. In contrast, neurons within the frontal cortex, the occipital cortex, and the cerebellum remain largely intact and preserved from neurodegenerative processes in AD. Brain tissues from the frontal cortex (F), the temporal cortex (T), and the hippocampus (H) of AD patients and healthy, age-matched control individuals were used for the herein disclosed examples. For illustrative purposes, the image of a normal healthy brain was taken from a publication by Strange (Brain Biochemistry and Brain Disorders, Oxford University Press, Oxford, 1992, p.4).

Figure 2 discloses the initial identification of a differential expression of the human gene coding for Ensadin-0477 in a fluorescence differential display screen. The figure shows a clipping of a large preparative fluorescent differential display gel. PCR products from the frontal cortex (F) and the temporal cortex (T) of two healthy control subjects and six AD patients were loaded in duplicate onto a denaturing polyacrylamide gel (from left to right). PCR products were obtained by amplification of the individual cDNAs with the corresponding one-base-anchor oligonucleotide and the specific Cy3 labelled random primers. The arrow indicates the migration position where significant differences in intensity of the signals for human Ensadin-0477 transcript derived from frontal cortex as compared to the signals derived from the temporal cortex of AD patients exist. The differential expression reflects an up-regulation of human Ensadin-0477 gene transcription in the temporal cortex versus the frontal cortex of AD patients. Comparing the signals derived from temporal cortex and frontal cortex of healthy non-AD control subjects with each other, no difference in signal intensity, i.e. no altered expression level can be detected.

Figure 3 and 4 illustrate the verification of the differential expression of the human Ensadin-0477 gene in AD brain tissues by quantitative RT-PCR analysis. Quantification of RT-PCR products from RNA samples collected from the frontal cortex (F) and the temporal cortex (T) of AD patients (Figure 3a) and samples from the frontal cortex (F) and the hippocampus (H) of AD patients (Figure 4a) was performed by the LightCycler rapid thermal cycling technique. Likewise, samples of healthy, age-matched control individuals were compared (Figure 3b for frontal cortex and temporal cortex, Figure 4b for frontal cortex and hippocampus). The data were normalized to the combined average values of a set of standard genes which showed no significant differences in their gene expression levels. Said set of standard genes consisted of genes for cyclophilin B, the ribosomal protein S9, the transferrin receptor, GAPDH, and beta-actin. The figures depict the kinetics of amplification by plotting the cycle number against the amount of amplified material as measured by its fluorescence. Note that the amplification kinetics of Ensadin-0477 cDNAs from both, the frontal and temporal cortices of a normal control individual, and from the frontal cortex and hippocampus of a normal control individual, respectively, during the exponential phase of the reaction are juxtaposed (Figures 3b and 4b, arrowhead), whereas in Alzheimer's disease (Figures 3a and 4a, arrowhead) there is a significant separation of the corresponding curves, indicating a differential expression of the gene coding for Ensadin-0477 in the respective analyzed brain regions.

Figure 5 discloses SEQ ID NO. 1; the amino acid sequence of the Ensadin-0477 protein. The full length human Ensadin-0477 protein comprises 937 amino acids. Amino acids 45 to 143 represent an ATP-binding domain, ATPase-like.

Figure 6 shows SEQ ID NO. 2, the nucleotide sequence of the human Ensadin-0477 cDNA, comprising 3832 nucleotides.

Figure 7 depicts SEQ ID NO. 3, the nucleotide sequence of the 259 bp Ensadin-0477 cDNA fragment, identified and obtained by fluorescence differential display and subsequent cloning.

Figure 8 outlines the sequence alignment of SEQ ID NO. 3 to the nucleotide sequence of the human cDNA clone dJ75H8.2, derived from GenBank accession number AL158821.

Figure 9 charts the schematic alignment of SEQ ID NO. 3 with Ensadin-0477 cDNA. The open rectangle represents the Ensadin-0477 open reading frame, thin bars represent the 5' and 3' untranslated regions (UTRs).

Table 1 lists the gene expression levels in the temporal cortex relative to the frontal cortex for the Ensadin-0477 gene in seven AD patients, herein identified by internal reference numbers P010, P011, P012, P014, P016, P017, P019 (0.85 to 4.60 fold) and five healthy, age-matched control individuals, herein identified by internal reference numbers C005, C008, C011, C012, C014 (0.78 to 1.27 fold). The scatter diagram visualizes individual values of the temporal to frontal cortex regulation ratios in control samples (dots) and in AD patient samples (triangles), respectively.

Table 2 lists the gene expression levels in the hippocampus relative to the frontal cortex for the Ensadin-0477 gene in six Alzheimer's disease patients, herein identified by internal reference numbers P010, P011, P012, P014, P016, P019 (1.29 to 2.49 fold) and three healthy, age-matched control individual, herein identified by internal reference numbers C004, C005, C008 (0.86 to 1.74 fold). The scatter diagram visualizes individual values of the hippocampus to frontal cortex regulation ratios in control samples (dots) and in AD patient samples (triangles).

EXAMPLE I:

(i) Brain tissue dissection from patients with AD:

Brain tissues from AD patients and age-matched control subjects were collected within 6 hours post-mortem and immediately frozen on dry ice. Sample sections from each tissue were fixed in paraformaldehyde for histopathological confirmation of the diagnosis. Brain areas for differential expression analysis were identified (see Figure 1) and stored at -80°C until RNA extractions were performed.

(ii) Isolation of total mRNA:

Total RNA was extracted from post-mortem brain tissue by using the RNeasy kit (Qiagen) according to the manufacturer's protocol. The accurate RNA concentration and the RNA quality were determined with the DNA LabChip system using the Agilent 2100 Bioanalyzer (Agilent Technologies). For additional quality testing of the prepared RNA, i.e. exclusion of partial degradation and testing for DNA contamination, specifically designed intronic GAPDH oligonucleotides and genomic DNA as reference control were used to generate a melting curve with the LightCycler technology as described in the manufacturer's protocol (Roche).

(iii) cDNA synthesis and identification of differentially expressed genes by fluorescence differential display (FDD):

In order to identify changes in gene expression in different tissues we employed a modified and improved differential display (DD) screening method. The original DD screening method is known to those skilled in the art (Liang and Pardee, Science 1995, 267: 1186-7). This technique compares two populations of RNA and provides clones of genes that are expressed in one population but not in the other. Several samples can be analyzed simultaneously and both up- and down-regulated genes can be identified in the same experiment. By adjusting and refining several steps in the DD method as well as modifying technical parameters, e.g. increasing redundancy, evaluating optimized reagents and conditions for reverse transcription of total RNA, optimizing polymerase chain reactions (PCR) and separation of the products thereof, a technique was developed which allows for highly reproducible and sensitive results. The applied and improved DD technique was described in detail by von der Kammer et al. (Nucleic Acids Research 1999, 27: 2211-2218). A set of 64 specifically designed random primers were developed (standard set) to achieve a statistically comprehensive analysis of all possible RNA species. Further, the method was modified to generate a preparative DD slab-gel technique, based on the use of fluorescently labelled primers. In the present invention, RNA populations from carefully selected post-mortem brain tissues (frontal cortex, temporal cortex, hippocampus) of AD patients and age-matched control subjects were compared.

As starting material for the DD analysis we used total RNA, extracted as described above (ii). Equal amounts of 0.05 μg RNA each were transcribed into cDNA in 20 μl reactions containing 0.5 mM each dNTP, 1 μl Sensiscript Reverse Transcriptase and 1x RT buffer (Qiagen), 10 U RNase inhibitor (Qiagen) and 1 μM of either one-base-anchor oligonucleotides $HT_{11}A$, $HT_{11}G$ or $HT_{11}C$ (Liang et al., Nucleic Acids Research 1994, 22:5763-5764; Zhao et al., Biotechniques 1995, 18:842-850). Reverse transcription was performed for 60 min at 37 °C with a final denaturation step at 93 °C for 5 min. 2 μl of the obtained cDNA each was subjected to a polymerase chain reaction (PCR) employing the corresponding one-base-anchor oligonucleotide (1 μM) along with either one of the Cy3 labelled random DD primers (1 μM), 1x GeneAmp PCR buffer (Applied Biosystems), 1.5 mM $MgCl_2$ (Applied Biosystems), 2 μM dNTP-Mix (dATP, dGTP, dCTP, dTTP Amersham Pharmacia Biotech), 5 % DMSO (Sigma), 1 U AmpliTaq DNA Polymerase (Applied Biosystems) in a 20 μl final volume. PCR conditions were set as follows: one round at 94 °C for 30 sec for denaturing, cooling 1 °C/sec down to 40 °C, 40 °C for 4 min for low-stringency annealing of primer, heating 1 °C/sec up to 72 °C, 72 °C for 1 min for extension. This round was followed by 39 high-stringency cycles: 94 °C for 30 sec, cooling 1 °C/sec down to 60 °C, 60 °C for 2 min, heating 1 °C/sec up to 72 °C, 72 °C for 1 min. One final step at 72 °C for 5 min was added to the last cycle (PCR cycler: Multi Cycler PTC 200, MJ Research). 8 μl DNA loading buffer were added to the 20 μl PCR product preparation, denatured for 5 min and kept on ice until loading onto a gel. 3.5 μl each were separated on 0.4 mm thick, 6 %-polyacrylamide (Long Ranger)/ 7 M urea sequencing gels in a slab-gel system (Hitachi Genetic Systems) at 2000 V, 60W, 30 mA, for 1 h 40 min. Following completion of the electrophoresis, gels were scanned with a FMBIO II fluorescence-scanner (Hitachi Genetic Systems), using the appropriate FMBIO II Analysis 8.0 software. A full-scale picture was printed, differentially expressed bands marked, excised from the gel, transferred into 1.5 ml containers, overlayed with 200 μl sterile water and kept at -20°C until extraction.

Elution and reamplification of DD products: The differential bands were extracted from the gel by boiling in 200 μl H2O for 10 min, cooling down on ice and precipitation from the supernatant fluids by using ethanol (Merck) and glycogen/sodium acetate (Merck) at - 20 °C over night, and subsequent centrifugation at 13.000 rpm for 25 min at 4 °C. Pellets were washed twice in ice-cold ethanol (80%), resuspended in 10 mM Tris pH 8.3 (Merck) and dialysed against 10 % glycerol (Merck) for 1 h at room temperature on a 0.025 μm VSWP membrane (Millipore). The obtained preparations were used as templates for reamplification by 15 high-stringency cycles in 25-μl PCR mixtures containing the corresponding primer pairs as used for the DD PCR (see above) under identical conditions, with the exception of the initial round at 94 °C for 5 min, followed by 15 cycles of: 94 °C for 45 sec, 60 °C for 45 sec, ramp 1 °C/sec to 70 °C for 45 sec, and one final step at 72 °C for 5 min. All oligonucleotide primers/probes as noted herein and below, were designed using the Primer Express Software v2.0 (Applied Biosystems). For the detection and verification of multiple isoforms of one gene, they were designed in a fashion that one primer/probe solely recognizes the flanking regions of one specifically spliced isoform.

Cloning and sequencing of DD products: Re-amplified cDNAs were analyzed with the DNA LabChip system (Agilent 2100 Bioanalyzer, Agilent Technologies) and ligated into the pCR-Blunt II-TOPO vector and transformed into *E.coli* Top10F' cells (Zero Blunt TOPO PCR Cloning Kit, Invitrogen) according to the manufacturer's instructions. Cloned cDNA fragments were sequenced by commercially available sequencing facilities. The result of one such FDD experiment for the Ensadin-0477 gene is shown in Figure 2.

(iv) Confirmation of differential expression by quantitative RT-PCR:

Positive corroboration of differential expression of the gene coding for Ensadin-0477 was performed using the LightCycler technology (Roche). This technique features rapid thermal cyling for the polymerase chain reaction as well as real-time measurement of fluorescent signals during amplification and therefore allows for highly accurate quantification of RT-PCR products by using a kinetic, rather than an endpoint readout. The ratios of Ensadin-0477 cDNA from the temporal cortex and frontal cortex, and from the hippocampus and frontal cortex, respectively, were determined (relative quantification).

First, a standard curve was generated to determine the efficiency of the PCR with specific primers for the gene coding for Ensadin-0477:

5'-CCACACCAGAAATGGGACCT-3' and

5'-AACACTCACTGACAGTGTGGCC-3'.

PCR amplification (95 °C and 1 sec, 56 °C and 5 sec, and 72 °C and 5 sec) was performed in a volume of 20 μl containing LightCycler-FastStart DNA Master SYBR Green I mix (contains FastStart Taq DNA polymerase, reaction buffer, dNTP mix with dUTP instead of dTTP, SYBR Green I dye, and 1 mM $MgCl_2$; Roche), 0.5 μM primers, 2 μl of a cDNA dilution series (final concentration of 40, 20, 10, 5, 1 and 0.5 ng human total brain cDNA; Clontech) and, depending on the primers used, additional 3 mM $MgCl_2$. Melting curve analysis revealed a single peak at approximately 79.5°C with no visible primer dimers. Quality and size of the PCR product were determined with the DNA LabChip system (Agilent 2100 Bioanalyzer, Agilent Technologies). A single peak at the expected size of 67 bp for the Ensadin-0477 gene was observed in the electropherogram of the sample.

In an analogous manner, the PCR protocol was applied to determine the PCR efficiency of a set of reference genes which were selected as a reference standard for quantification. In the present invention, the mean value of five such reference genes was determined: (1) cyclophilin B, using the specific primers 5'-ACTGAAGCACTACGGGCCTG-3' and 5'-AGCCGTTGGTGTCTT-TGCC-3' except for $MgCl_2$ (an additional 1 mM was added instead of 3 mM). Melting curve analysis revealed a single peak at approximately 87 °C with no visible primer dimers. Agarose gel analysis of the PCR product showed one single band of the expected size (62 bp). (2) Ribosomal protein S9 (RPS9), using the specific primers 5'-GGTCAAATTTACCCTGGCCA-3' and 5'-TCTCATCAAGCGTCAGCAGTTC-3' (exception: additional 1 mM $MgCl_2$ was added instead of 3 mM). Melting curve analysis revealed a single peak at approximately 85°C with no visible primer dimers. Agarose gel analysis of the PCR product showed one single band with the expected size (62 bp). (3) beta-actin, using the specific primers 5'-TGGAACGGTGAAGGTGACA-3' and 5'-GGCAAGGGACTTCCTGTAA-3'. Melting curve analysis revealed a single peak at approximately 87°C with no visible primer dimers. Agarose gel analysis of the PCR product showed one single band with the expected size (142 bp). (4) GAPDH, using the specific primers 5'-CGTCATGGGTGTGAACCATG-3' and 5'-GCTAAGCAGTTGGTGGTGCAG-3'. Melting curve analysis revealed a single peak at approximately 83°C with no visible primer dimers. Agarose gel analysis of the PCR product showed one single band with the expected size (81 bp). (5) Transferrin receptor TRR, using the specific primers 5'-GTCGCTGGTCAGTTCGTGATT-3' and 5'-AGCAGTTGGCTGTTGTACCTCTC-3'. Melting curve analysis revealed a single peak at approximately 83°C with no visible primer dimers. Agarose gel analysis of the PCR product showed one single band with the expected size (80 bp).

For calculation of the values, first the logarithm of the cDNA concentration was plotted against the threshold cycle number $C_t$ for Ensadin-0477 and the five reference standard genes. The slopes and the intercepts of the standard curves (i.e. linear regressions) were calculated for all genes. In a second step, cDNAs from temporal cortex and frontal cortex, and from hippocampus and frontal cortex, respectively, were analyzed in parallel and normalized to cyclophilin B. The $C_t$ values were measured and converted to ng total brain cDNA using the corresponding standard curves:

$$10 \wedge ((C_t \text{ value - intercept}) / \text{slope}) \qquad [\text{ng total brain cDNA}]$$

The values for temporal and frontal cortex Ensadin-0477 cDNAs, and the values for hippocampus and frontal cortex Ensadin-0477 cDNAs, respectively, were normalized to cyclophilin B and the ratios were calculated according to formulas:

$$\text{Ratio} = \frac{\text{Ensadin-0477 temporal [ng] / cyclophilin B temporal [ng]}}{\text{Ensadin-0477 frontal [ng] / cyclophilin B frontal [ng]}}$$

$$\text{Ratio} = \frac{\text{Ensadin-0477 hippocampus [ng] / cyclophilin B hippocampus [ng]}}{\text{Ensadin-0477 frontal [ng] / cyclophilin B frontal [ng]}}$$

In a third step, the set of reference standard genes was analyzed in parallel to determine the mean average value of the temporal to frontal ratios, and of the hippocampal to frontal ratios, respectively, of expression levels of the reference standard genes for each individual brain sample. As cyclophilin B was analyzed in step 2 and step 3, and the ratio from one gene to another gene remained constant in different runs, it was possible to normalize the values for Ensadin-0477 to the mean average value of the set of reference standard genes instead of normalizing to one single gene alone. The calculation was performed by dividing the respective ratio shown above by the deviation of cyclophilin B from the mean value of all housekeeping genes. The results of such quantitative RT-PCR analysis for the Ensadin-0477 gene are shown in Figure 3 and 4.

**Claims**

1. A method of diagnosing or prognosticating a neurodegenerative disease in a subject, or determining whether a subject is at increased risk of developing said disease, comprising:

   determining a level and/or an activity of

   (i) a transcription product of a gene having the nucleotide sequence of SEQ ID No. 2, and/or
   (ii) a translation product of a gene having the nucleotide sequence of SEQ ID No. 2,

   in a sample from said subject and comparing said level and/or said activity to a reference value representing a known disease or health status, thereby diagnosing or prognosticating said neurodegenerative disease in said subject, or determining whether said subject is at increased risk of developing said neurodegenerative disease.

2. A method of monitoring the progression of a neurodegenerative disease in a subject, comprising:

   determining a level and/or an activity of

   (i) a transcription product of a gene having the nucleotide sequence of SEQ ID No. 2, and/or
   (ii) a translation product of a gene having the nucleotide sequence of SEQ ID No. 2,

   in a series of samples taken from said subject over a period of time and comparing said level and/or said activity to a reference value representing a known disease or health status, thereby monitoring the progression of said neurodegenerative disease in said subject.

3. A method of evaluating a treatment for a neurodegenerative disease, comprising:

   determining a level and/or an activity of

   (i) a transcription product of a gene having the nucleotide sequence of SEQ ID No. 2, and/or
   (ii) a translation product of a gene having the nucleotide sequence of SEQ ID No. 2,

   in samples taken from said subject before and after being treated for said disease and comparing said level

and/or said activity to a reference value representing a known disease or health status, thereby evaluating said treatment for said neurodegenerative disease.

**4.** The method according to any of claims 1 to 3 wherein said neurodegenerative disease is Alzheimer's disease.

**5.** The method according to any of claims 1 to 4 wherein said sample comprises a cell, or a tissue, or a body fluid, in particular cerebrospinal fluid or blood.

**6.** The method according to any of claims 1 to 5 wherein said reference value is that of a level and/or an activity of

(i) a transcription product of a gene having the nucleotide sequence of SEQ ID No. 2, and/or
(ii) a translation product of a gene having the nucleotide sequence of SEQ ID No. 2,

in a sample from a subject not suffering from said neurodegenerative disease.

**7.** The method according to any of claims 1 to 6 wherein an alteration in the level and/or activity of a transcription product of a gene having the nucleotide sequence of SEQ ID No. 2 and/or of a translation product of a gene having the nucleotide sequence of SEQ ID No. 2 in a sample cell, or tissue, or body fluid, in particular cerebrospinal fluid, from said subject relative to a reference value representing a known health status indicates a diagnosis, or prognosis, or increased risk of Alzheimer's disease in said subject.

**8.** Use of a kit for diagnosing or prognosticating a neurodegenerative disease, in particular Alzheimer's disease, in a sample of a subject, or determining the propensity or predisposition of a subject to develop such a disease, said kit comprising at least one reagent which is selected from the group consisting of (i) reagents that selectively detect a transcription product of a gene having the nucleotide sequence of SEQ ID No. 2 and (ii) reagents that selectively detect a translation product of a gene having the nucleotide sequence of SEQ ID No. 2,
said use being effected by:
(i) detecting a level, or an activity, or both said level and said activity, of said transcription product and/or said translation product of a gene having the nucleotide sequence of SEQ ID No. 2 in a sample from said subject; and
(ii) diagnosing or prognosticating a neurodegenerative disease, in particular Alzheimer's disease, or determining the propensity or predisposition of said subject to develop such a disease, wherein a varied level, or activity, or both said level and said activity, of said transcription product and/or said translation product compared to a reference value representing a known health status; or a level, or activity, or both said level and said activity, of said transcription product and/or said translation product similar or equal to a reference value representing a known disease status indicates a diagnosis or prognosis of a neurodegenerative disease, in particular Alzheimer's disease, or an increased propensity or predisposition of developing such a disease.

**9.** A recombinant mouse comprising a non-native gene sequence having the nucleotide sequence of SEQ ID No. 2 said mouse being obtainable by:

(i) providing a gene targeting construct comprising said gene sequence and a selectable marker sequence, and
(ii) introducing said targeting construct into a stem cell of a mouse, and
(iii) introducing said mouse stem cell into a mouse embryo, and
(iv) transplanting said embryo into a pseudopregnant mouse, and
(v) allowing said embryo to develop to term, and
(vi) identifying a genetically altered mouse whose genome comprises a modification of said gene sequence in both alleles, and breeding the genetically altered mouse of step (vi) to obtain a genetically altered mouse whose genome comprises a modification of said endogenous gene, wherein said disruption results in said mouse exhibiting a predisposition to developing symptoms of a neurodegenerative disease or related diseases or disorders.

**10.** Use of the recombinant mouse according to claim 9 for testing and validating compounds, agents, and modulators in the development of diagnostics for neurodegenerative diseases, in particular for Alzheimer's disease.

**11.** A protein molecule, said protein molecule having the amino acid sequence of SEQ ID No. 1.

**12.** A nucleic acid molecule having the nucleotide sequence of SEQ ID NO.2.

13. An antibody specifically immunoreactive with an immunogen, wherein said immunogen is a protein having the amino acid sequence of SEQ ID NO. 1.

14. Use of an antibody of claim 13 for detecting the pathological state of a cell in a sample from a subject, comprising immunocytochemical staining of said cell with said antibody, wherein an altered degree of staining, or an altered staining pattern in said cell compared to a cell representing a known health status indicates a pathological state of said cell which pathological state relates to a neurodegenerative disease.

## Patentansprüche

1. Verfahren zum Diagnostizieren oder Prognostizieren einer neurodegenerativen Krankheit bei einem Patienten oder zum Bestimmen, ob ein Patient ein erhöhtes Risiko hat, diese Krankheit zu entwickeln, umfassend:

   Bestimmen einer Konzentration und/oder einer Aktivität von:

   (i) einem Transcriptionsprodukt eines Gens mit der Nucleotidsequenz von SEQ ID Nr. 2; und/oder
   (ii) einem Translationsprodukt eines Gens mit der Nucleotidsequenz von SEQ ID Nr. 2;

   in einer von dem Patienten stammenden Probe und Vergleichen der Konzentration und/oder der Aktivität mit einem Referenzwert, der einen bekannten Krankheits- oder Gesundheitszustand darstellt, wodurch die neurodegenerative Krankheit bei dem Patienten diagnostiziert oder prognostiziert wird oder bestimmt wird, ob der Patient ein erhöhtes Risiko hat, die neurodegenerative Krankheit zu entwickeln.

2. Verfahren zur Überwachung des Fortschritts einer neurodegenerativen Krankheit bei einem Patienten, umfassend:

   Bestimmen einer Konzentration und/oder einer Aktivität von:

   (i) einem Transcriptionsprodukt eines Gens mit der Nucleotidsequenz von SEQ ID Nr. 2; und/oder
   (ii) einem Translationsprodukt eines Gens mit der Nucleotidsequenz von SEQ ID Nr. 2;
   in einer Reihe von Proben, die über einen Zeitraum hinweg von dem Patienten entnommen wurden, und Vergleichen der Konzentration und/oder der Aktivität mit einem Referenzwert, der einen bekannten Krankheits- oder Gesundheitszustand darstellt, wodurch der Fortschritt der neurodegenerativen Krankheit bei dem Patienten überwacht wird.

3. Verfahren zur Bewertung einer Behandlung auf eine neurodegenerative Krankheit, umfassend:

   Bestimmen einer Konzentration und/oder einer Aktivität von:

   (i) einem Transcriptionsprodukt eines Gens mit der Nucleotidsequenz von SEQ ID Nr. 2; und/oder
   (ii) einem Translationsprodukt eines Gens mit der Nucleotidsequenz von SEQ ID Nr. 2;
   in Proben, die von dem Patienten erhalten wurden, bevor und nachdem er auf die Krankheit behandelt wurde, und Vergleichen der Konzentration und/oder der Aktivität mit einem Referenzwert, der einen bekannten Krankheits- oder Gesundheitszustand darstellt, wodurch die Behandlung auf die neurodegenerative Krankheit bewertet wird.

4. Verfahren gemäß einem der Ansprüche 1 bis 3, wobei es sich bei der neurodegenerativen Krankheit um die Alzheimer-Krankheit handelt.

5. Verfahren gemäß einem der Ansprüche 1 bis 4, wobei die Probe eine Zelle oder ein Gewebe oder eine Körperflüssigkeit, insbesondere Liquor oder Blut, umfasst.

6. Verfahren gemäß einem der Ansprüche 1 bis 5, wobei der Referenzwert der Referenzwert einer Konzentration und/oder einer Aktivität von

   (i) einem Transcriptionsprodukt eines Gens mit der Nucleotidsequenz von SEQ ID Nr. 2; und/oder
   (ii) einem Translationsprodukt eines Gens mit der Nucleotidsequenz von SEQ ID Nr. 2;
   in einer Probe von einem Patienten, der nicht an der neurodegenerativen Krankheit leidet, ist.

7. Verfahren gemäß einem der Ansprüche 1 bis 6, wobei eine Veränderung in der Konzentration und/oder Aktivität eines Transcriptionsprodukts eines Gens mit der Nucleotidsequenz von SEQ ID Nr. 2 und/oder eines Translationsprodukts eines Gens mit der Nucleotidsequenz von SEQ ID Nr. 2 in einer Zell- oder Gewebe- oder Körperflüssigkeitsprobe, insbesondere Liquorprobe, von dem Patienten relativ zu einem Referenzwert, der einen bekannten Gesundheitszustand darstellt, eine Diagnose oder Prognose oder ein erhöhtes Risiko von Alzheimer-Krankheit bei dem Patienten anzeigt.

8. Verwendung eines Kits zum Diagnostizieren oder Prognostizieren einer neurodegenerativen Krankheit, insbesondere Alzheimer-Krankheit, in einer von einem Patienten stammenden Probe oder zum Bestimmen der Neigung oder Prädisposition eines Patienten, eine solche Krankheit zu entwickeln, wobei der Kit wenigstens ein Reagens umfasst, das aus der Gruppe ausgewählt ist, die aus Folgendem besteht: (i) Reagentien, die selektiv ein Transcriptionsprodukt eines Gens mit der Nucleotidsequenz von SEQ ID Nr. 2 nachweisen, und (ii) Reagentien, die selektiv ein Translationsprodukt eines Gens mit der Nucleotidsequenz von SEQ ID Nr. 2 nachweisen;
wobei die Verwendung realisiert wird durch:

   (i) Nachweisen einer Konzentration oder einer Aktivität oder sowohl einer Konzentration als auch einer Aktivität eines Transcriptionsprodukts und/oder eines Translationsprodukts eines Gens mit der Nucleotidsequenz von SEQ ID Nr. 2 in einer von dem Patienten stammenden Probe und (ii) Diagnostizieren oder Prognostizieren einer neurodegenerativen Krankheit, insbesondere Alzheimer-Krankheit, oder Bestimmen der Neigung oder Prädisposition des Patienten, eine solche Krankheit zu entwickeln, wobei eine variierte Konzentration oder Aktivität oder sowohl Konzentration als auch Aktivität des Transcriptionsprodukts und/oder des Translationsprodukts im Vergleich zu einem Referenzwert, der einen bekannten Gesundheitszustand darstellt, oder eine Konzentration oder Aktivität oder sowohl Konzentration als auch Aktivität des Transcriptionsprodukts und/oder Translationsprodukts, die einem Referenzwert, der einen bekannten Krankheitszustand darstellt, ähnlich oder gleich sind, eine Diagnose oder Prognose einer neurodegenerativen Krankheit, insbesondere Alzheimer-Krankheit, oder eine erhöhte Neigung oder Prädisposition, eine solche Krankheit zu entwickeln, anzeigt.

9. Rekombinante Maus, die eine nichtnative Gensequenz mit der Nucleotidsequenz von SEQ ID Nr. 2 umfasst, wobei die Maus erhältlich ist durch:

   (i) Bereitstellen eines Genzielsteuerungskonstrukts, das die Gensequenz und eine Selektionsmarkersequenz umfasst; und
   (ii) Einführen des Zielsteuerungskonstrukts in eine Stammzelle einer Maus; und
   (iii) Einführen der Mäusestammzelle in einen Mäuseembryo; und
   (iv) Transplantieren des Embryos in eine pseudoträchtige Maus; und
   (v) Entwickelnlassen des Embryos bis zur Reife; und
   (vi) Identifizieren einer genetisch veränderten Maus, deren Genom eine Modifikation der Gensequenz in beiden Allelen umfasst; und

Aufziehen der genetisch veränderten Maus von Schritt (vi), so dass man eine genetisch veränderte Maus erhält, deren Genom eine Modifikation des endogenen Gens umfasst, wobei der Eingriff dazu führt, dass die Maus eine Prädisposition aufweist, Symptome einer neurodegenerativen Krankheit oder verwandter Krankheiten oder Störungen zu entwickeln.

10. Verwendung der rekombinanten Maus gemäß Anspruch 9 zum Testen und Bewerten von Verbindungen, Mitteln und Modulatoren bei der Entwicklung von Diagnostika für neurodegenerative Krankheiten, insbesondere für die Alzheimer-Krankheit.

11. Proteinmolekül, wobei das Proteinmolekül die Aminosäuresequenz von SEQ ID Nr. 1 aufweist.

12. Nucleinsäuremolekül mit der Nucleotidsequenz von SEQ ID Nr. 2.

13. Antikörper, der gegenüber einem Immunogen spezifisch immunreaktiv ist,
wobei das Immunogen ein Protein mit der Aminosäuresequenz von SEQ ID Nr. 1 ist.

14. Verwendung eines Antikörpers gemäß Anspruch 13 zum Nachweisen des pathologischen Zustands einer Zelle in einer von einem Patienten stammenden Probe, umfassend das immunocytochemische Anfärben der Zelle mit dem Antikörper, wobei ein veränderter Grad der Anfärbung oder ein verändertes Anfärbungsmuster in der Zelle im Vergleich zu einer Zelle, die einen bekannten Gesundheitszustand repräsentiert, einen pathologischen Zustand der Zelle anzeigt, wobei sich der pathologische Zustand auf eine neurodegenerative Krankheit bezieht.

## Revendications

**1.** Procédé permettant de diagnostiquer ou de pronostiquer une maladie neurodégénérative chez un sujet, ou de déterminer si un sujet présente un risque accru de développer ladite maladie, comprenant la :

détermination du niveau et/ou de l'activité :

(i) d'un produit de transcription d'un gène ayant la séquence de nucléotides SEQ ID N° 2 et/ou
(ii) d'un produit de traduction d'un gène ayant la séquence de nucléotides SEQ ID N° 2,

dans un échantillon provenant dudit sujet, et comparer ledit niveau et/ou ladite activité à une valeur de référence représentant une maladie ou un état de santé connu, diagnostiquant ou pronostiquant ainsi ladite maladie neurodégénérative chez ledit sujet, ou déterminant si ledit sujet présente un risque accru de développer ladite maladie neurodégénérative.

**2.** Procédé de suivi de la progression d'une maladie neurodégénérative chez un sujet, comprenant la :

détermination du niveau et/ou de l'activité :

(i) d'un produit de transcription d'un gène ayant la séquence de nucléotides SEQ ID N° 2 et/ou
(ii) d'un produit de traduction d'un gène ayant la séquence de nucléotides SEQ ID N° 2,

dans une série d'échantillons prélevés chez ledit sujet pendant une certaine période, et comparer ledit niveau et/ou ladite activité à une valeur de référence représentant une maladie ou un état de santé connu, suivant ainsi la progression de ladite maladie neurodégénérative chez ledit sujet.

**3.** Procédé d'évaluation d'un traitement d'une maladie neurodégénérative, comprenant la :

détermination du niveau et/ou de l'activité :

(i) d'un produit de transcription d'un gène ayant la séquence de nucléotides SEQ ID N° 2 et/ou
(ii) d'un produit de traduction d'un gène ayant la séquence de nucléotides SEQ ID N° 2,

dans des échantillons prélevés chez ledit sujet avant et après que celui-ci ne soit traité pour ladite maladie, et comparer ledit niveau et/ou ladite activité à une valeur de référence représentant une maladie ou un état de santé connu, évaluant ainsi ledit traitement de ladite maladie neurodégénérative.

**4.** Procédé selon l'une quelconque des revendications 1 à 3, dans lequel ladite maladie neurodégénérative est la maladie d'Alzheimer.

**5.** Procédé selon l'une quelconque des revendications 1 à 4, dans lequel ledit échantillon comprend une cellule ou un tissu ou un fluide corporel, en particulier le liquide céphalorachidien ou le sang.

**6.** Procédé selon l'une quelconque des revendications 1 à 5, dans lequel ladite valeur de référence est celle correspondant au niveau et/ou à l'activité :

(i) d'un produit de transcription d'un gène ayant la séquence de nucléotides SEQ ID N° 2 et/ou
(ii) d'un produit de traduction d'un gène ayant la séquence de nucléotides SEQ ID N° 2,

dans un échantillon provenant d'un sujet ne souffrant pas de ladite maladie neurodégénérative.

**7.** Procédé selon l'une quelconque des revendications 1 à 6, dans lequel une variation du niveau et/ou de l'activité d'un produit de transcription d'un gène ayant la séquence de nucléotides SEQ ID N° 2 et/ou d'un produit de traduction d'un gène ayant la séquence de nucléotides SEQ ID N° 2, dans une cellule, un tissu ou un fluide corporel échantillon, en particulier le liquide céphalorachidien, provenant dudit sujet, par rapport à une valeur de référence représentant un état de santé connu, indique un diagnostic ou un pronostic, ou un risque accru de maladie d'Alzheimer chez ledit sujet.

**8.** Utilisation d'une trousse permettant de diagnostiquer ou de pronostiquer une maladie neurodégénérative, en particulier la maladie d'Alzheimer, dans un échantillon d'un sujet, ou permettant de déterminer la tendance ou la prédisposition d'un sujet à développer une telle maladie, ladite trousse comprenant au moins un réactif qui est choisi dans le groupe formé par (i) des réactifs qui détectent sélectivement un produit de transcription d'un gène ayant la séquence de nucléotides SEQ ID N° 2 et (ii) des réactifs qui détectent sélectivement un produit de traduction d'un gène ayant la séquence de nucléotides SEQ ID N° 2, ladite utilisation étant réalisée en :

(i) détectant un niveau ou une activité, ou à la fois ledit niveau et ladite activité, dudit produit de transcription et/ou dudit produit de traduction d'un gène ayant la séquence de nucléotides SEQ ID N° 2 dans un échantillon provenant dudit sujet ; et (ii) en diagnostiquant ou en pronostiquant une maladie neurodégénérative, en particulier la maladie d'Alzheimer, ou en déterminant la tendance ou la prédisposition dudit sujet à développer une telle maladie, dans laquelle un niveau ou une activité, ou à la fois ledit niveau et ladite activité, dudit produit de transcription et/ou dudit produit de traduction, différent d'une valeur de référence représentant un état de santé connu ; ou un niveau ou une activité, ou à la fois ledit niveau et ladite activité, dudit produit de transcription et/ou dudit produit de traduction, similaire ou égal à une valeur de référence représentant un état pathologique connu, indique un diagnostic ou un pronostic d'une maladie neurodégénérative, en particulier la maladie d'Alzheimer, ou une tendance ou prédisposition accrue à développer une telle maladie.

**9.** Souris transgénique comprenant une séquence de gènes non native ayant la séquence de nucléotides SEQ ID N° 2, ladite souris pouvant être obtenus :

(i) en fournissant un produit de recombinaison ciblant un gène comprenant ladite séquence de gènes ainsi qu'une séquence marqueur sélectionnable, et
(ii) en introduisant ledit produit de recombinaison ciblant dans une cellule souche d'une souris, et
(iii)en introduisant ladite cellule souche de souris dans un embryon de souris, et
(iv) en transplantant ledit embryon dans une souris pseudogestante, et
(v) en laissant ledit embryon se développer jusqu'à terme, et
(vi) en identifiant une souris génétiquement modifiée dont le génome comprend une modification de ladite séquence de gènes au niveau des deux allèles, et en reproduisant la souris génétiquement modifiée de l'étape (vi) de façon à obtenir une souris génétiquement modifiée dont le génome comprend une modification dudit gène endogène, dans laquelle ladite perturbation fait que ladite souris présente une prédisposition au développement des symptômes d'une maladie neurodégénérative, ou de maladies ou de troubles apparentés.

**10.** Utilisation de la souris transgénique selon la revendication 9 pour tester et valider des composés, des agents et des modulateurs, dans la mise au point de diagnostics pour les maladies neurodégénératives, en particulier la maladie d'Alzheimer.

**11.** Molécule protéique, ladite molécule protéique ayant la séquence d'acides aminés SEQ ID N° 1.

**12.** Molécule d'acide nucléique ayant la séquence de nucléotides SEQ ID N° 2.

**13.** Anticorps spécifiquement immunoréactif envers un agent immunogène, dans lequel ledit agent immunogène est une protéine ayant la séquence d'acides aminés SEQ ID N° 1.

**14.** Utilisation d'un anticorps selon la revendication 13 pour détecter l'état pathologique d'une cellule dans un échantillon provenant d'un sujet, comprenant la coloration immunocytochimique de ladite cellule avec ledit anticorps, dans laquelle une variation du degré de coloration ou du schéma de coloration dans ladite cellule par rapport à une cellule représentant un état de santé connu indique un état pathologique de ladite cellule, lequel état pathologique est lié à une maladie neurodégénérative.

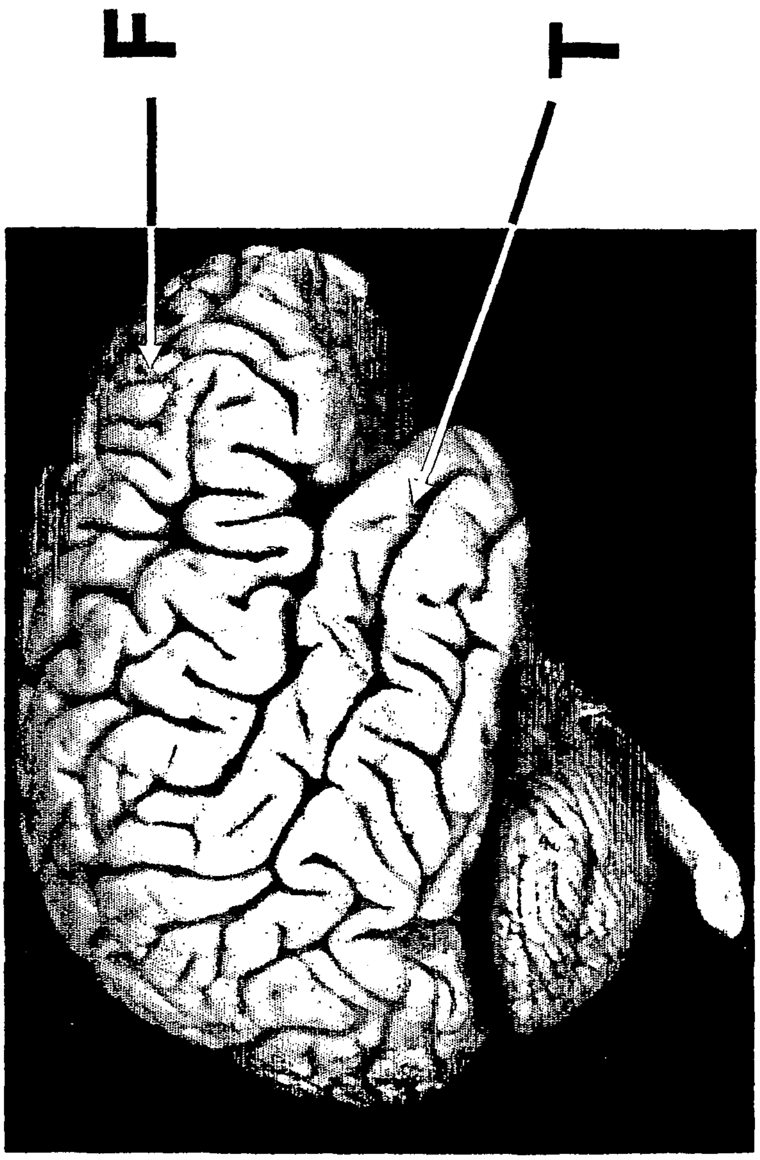


**Fig. 1: Identification of Genes Involved in Alzheimer's Disease Pathology**

Fig. 2: Identification of differentially expressed genes in a fluorescence differential display screen

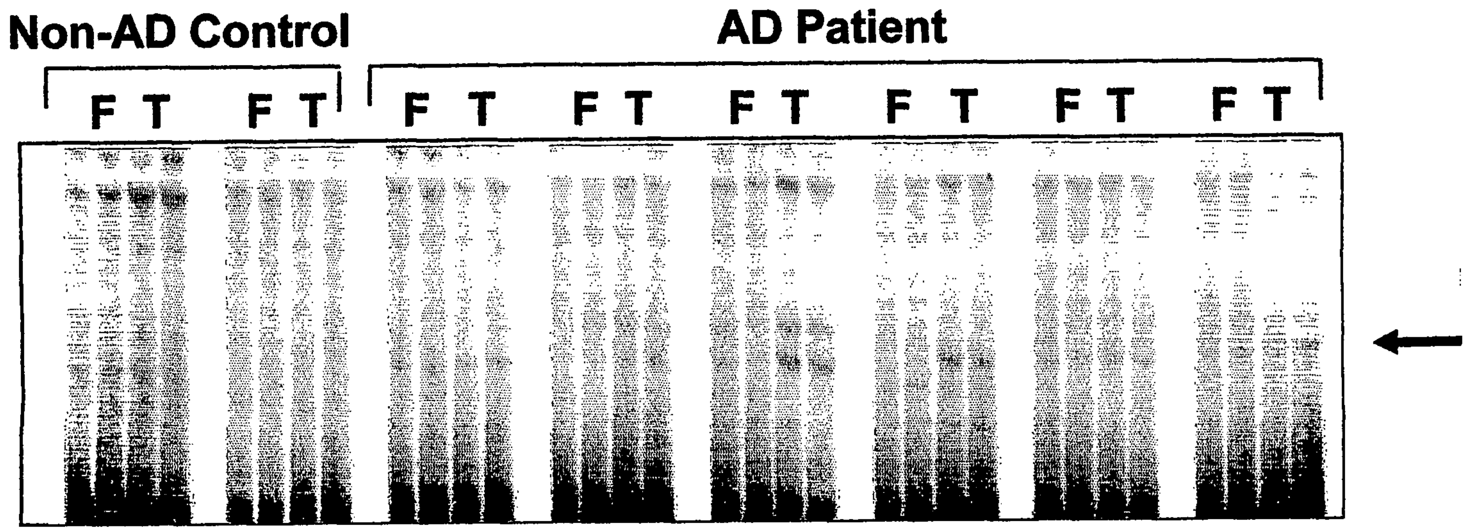

**Fig. 3: Verification of differential expression of Ensadin-0477 by quantitative RT-PCR**

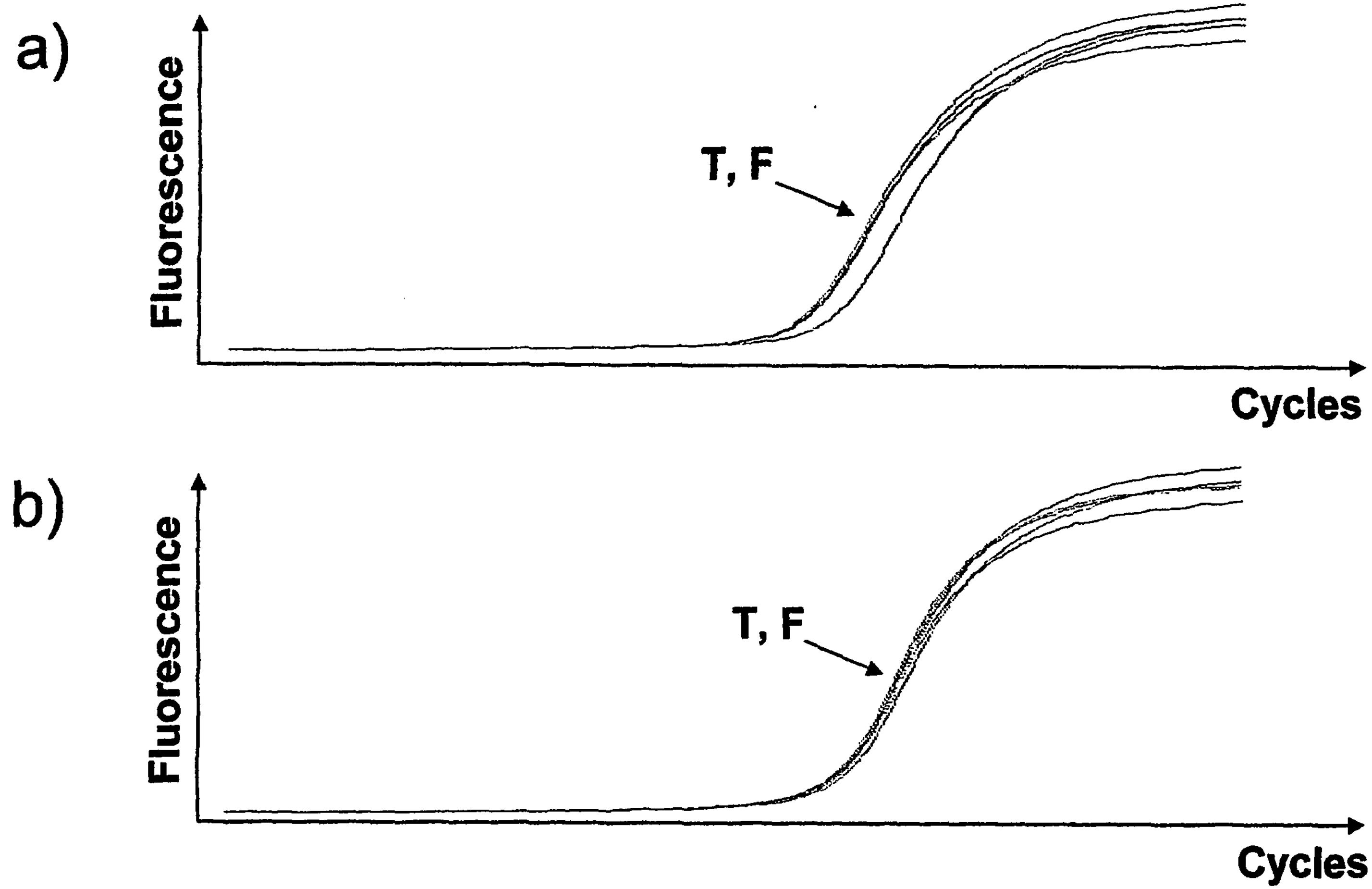

**Fig. 4: Verification of differential expression of Ensadin-0477 by quantitative RT-PCR**

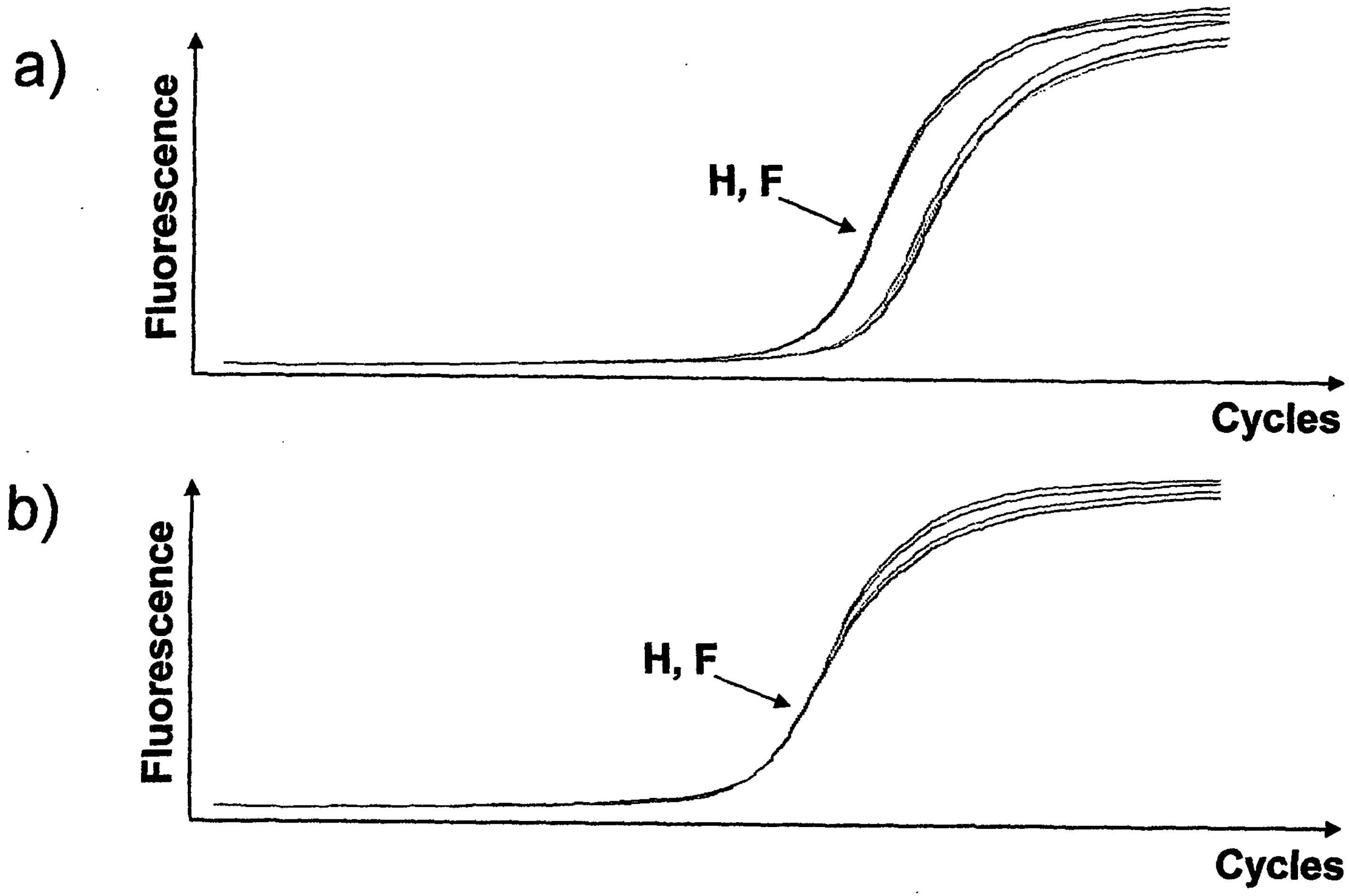

## Fig. 5: SEQ ID NO. 1: amino acid sequence of human Ensadin-0477

**Length: 937 aa**

```
  1  MLLYRGAPAG PGAPGCGLAR PGGGPQAFGI RLSTMSPRYL QSNSSSHTRP
 51  FSAIAELLDN AVDPDVSART VFIDVEEVKN KSCLTFTDDG CGMTPHKLHR
101  MLSFGFTDKV IKKSQCPIGV FGNGFKSGSM RLGKDALVFT KNGGTLTVGL
151  LSQTYLECVQ AQAVIVPIVP FNQQNKKMII TEDSLPSLEA ILNYSIFNRE
201  NDLLAQFDAI PGKKGTRVLI WNIRRNKNGK SELDFDTDQY DILVSDFDTE
251  EKMTGGVTSE LPETEYSLRA FCGILYMKPR MKIFLRQKKV TTQMIAKSLA
301  NVEYDTYKPT FTNKQVRITF GFSCKNSNQF GIMMYHNNRL IKSFEKVGCQ
351  VKPTRGEGVG VIGVIECNFL KPAYNKQDFE YTKEYRLTIN ALAQKLNAYW
401  KEKTSQDNFE TSTVARPIPK VPDQTWVQCD ECLKWRKLPG KIDPSMLPAR
451  WFCYYNSHPK YRRCSVPEEQ ELTDEDLCLS KAKKQEQTVE EKKKMPMENE
501  NHQVFSNPPK ILTVQEMAGL NNKTIGYEGI HSPSVLPSGG EESRSPSLQL
551  KPLDSSVLQF SSKYKWILGE EPVEKRRRLQ NEMTTPSLDY SMPAPYRRVE
601  APVAYPEGEN SHDKSSSERS TPPYLFPEYP EASKNTGQNR EVSILYPGAK
651  DQRQGSLLPE ELEDQMPRLV AEESNRGSTT INKEEVNKGP FVAVVGVAKG
701  VRDSGAPIQL IPFNREELAE RRKAVESWNP VPYSVASAAI PAAAIGEKAR
751  GYEESEGHNT PKLKNQRELE ELKRTTEKLE RVLAERNLFQ QKVEELEQER
801  NHWQSEFKKV QHELVIYSTQ EAEGLYWSKK HMGYRQAEFQ ILKAELERTK
851  EEKQELKEKL KETETHLEML QKAQVSYRTP EGDDLERALA KLTRLRIHVS
901  YLLTSVLPHL ELREIGYDSE QVDGILYTVL EANHILD*
```

# Fig. 6: SEQ ID NO. 2: nucleotide sequence of human Ensadin-0477 cDNA

**Length: 3832 bp**

```
   1 ATATTTCTGT ACGGCCCAGA GGCAGCCCGG GCAGTCGCCG GGTCGGTAGA
  51 GGTGCTGACA GGGCGGAGCC GCCGCTTGCC TCAGCCGGCC TCACCCCTCC
 101 CGCCCGGGAC AGGTGGCTGG AGCGCGCTCC GCCTCCGCCA GCTGCGAGTG
 151 GGAGCGAGGG AGGGCCGGTC CCGGACGGTC GCCGCCAGCT GCGAGTGGAA
 201 GTGAGCGAGG GCTAGTCCCG GACCGCCGGG CCAGGGGTCC GGCGGCAGCA
 251 GACGGGTACC GTGGCGGCCA AAAAAATGCT CCTGTACCGA GGGGCCCCCG
 301 CCGGCCCTGG CGCGCCGGGC TGCGGGCTGG CCCGGCCCGG CGGCGGCCCG
 351 CAGGCCTTCG GGATCCGCCT GAGCACGATG AGCCCCCGCT ACCTCCAGAG
 401 CAACTCCAGC AGCCACACGC GACCCTTCAG TGCCATCGCG GAGCTGCTAG
 451 ATAATGCTGT AGATCCAGAT GTATCTGCCA GGACGGTCTT TATAGATGTT
 501 GAGGAGGTCA AGAATAAATC TTGTTTGACC TTTACCGATG ATGGATGTGG
 551 GATGACACCT CATAAACTAC ACCGAATGCT CAGCTTTGGC TTTACAGATA
 601 AAGTAATAAA GAAGAGCCAG TGTCCCATTG GGGTCTTTGG TAATGGTTTC
 651 AAGTCAGGCT CCATGCGGCT AGGAAAGGAC GCCCTTGTCT TCACCAAGAA
 701 TGGGGGTACT CTCACTGTTG GACTTCTATC ACAGACCTAT CTGGAATGTG
 751 TCCAGGCCCA GGCAGTTATT GTACCAATTG TTCCATTCAA CCAGCAAAAC
 801 AAAAAAATGA TTATTACCGA GGATTCATTG CCCAGCCTAG AAGCCATCTT
 851 GAACTATTCC ATTTTCAACC GTGAAAATGA CCTGCTGGCC CAGTTTGATG
 901 CCATCCCAGG CAAAAAAGGC ACTCGTGTTC TCATTTGGAA CATCCGCAGA
 951 AATAAAAATG GAAAATCTGA GTTGGACTTT GATACAGATC AATATGACAT
1001 CCTGGTATCA GACTTTGACA CAGAAGAAAA AATGACTGGC GGTGTTACCT
1051 CTGAGCTACC AGAAACAGAA TATTCTTTAA GGGCATTTTG TGGTATTCTA
1101 TACATGAAGC CACGCATGAA AATTTTTCTG CGTCAAAAGA AGGTGACTAC
1151 CCAGATGATT GCCAAGAGCC TGGCCAATGT AGAATATGAT ACATATAAAC
1201 CTACCTTCAC AAATAAGCAG GTGAGAATCA CCTTTGGGTT CTCTTGCAAG
1251 AATAGTAACC AGTTTGGAAT AATGATGTAT CATAACAACC GACTCATAAA
1301 ATCTTTTGAG AAGGTGGGGT GCCAGGTGAA GCCAACTCGT GGAGAAGGTG
1351 TAGGAGTAAT TGGAGTCATT GAGTGCAATT TCCTAAAACC TGCCTACAAC
1401 AAACAAGACT TTGAGTATAC CAAGGAGTAC CGGCTAACAA TAAATGCCCT
1451 TGCCCAGAAG CTCAATGCTT ACTGGAAGGA AAAAACATCT CAAGATAATT
1501 TTGAGACCTC AACTGTAGCC AGGCCAATAC CGAAGGTTCC TGACCAGACA
1551 TGGGTTCAGT GTGATGAGTG TCTTAAATGG AGAAAGCTTC CTGGGAAGAT
1601 TGATCCATCC ATGTTACCTG CAAGATGGTT TTGTTATTAT AATTCCCATC
1651 CAAAGTACAG GAGATGCTCT GTTCCAGAGG AACAAGAACT CACTGATGAA
1701 GACCTGTGCT TGAGCAAAGC TAAGAAACAA GAACAAACTG TTGAGGAGAA
1751 GAAGAAGATG CCTATGGAAA ATGAGAACCA CCAGGTATTC AGTAATCCAC
1801 CAAAGATCCT TACTGTTCAA GAAATGGCTG GATTGAATAA CAAGACAATT
1851 GGATATGAGG GAATTCATAG CCCTAGTGTG CTTCCTTCTG GTGGAGAAGA
```

1901 AAGCAGATCA CCATCTCTTC AACTTAAGCC TCTGGATTCC AGTGTTTTAC
1951 AGTTTTCCAG TAAGTACAAA TGGATCCTAG GTGAAGAACC GGTGGAGAAA
2001 CGAAGAAGGC TCCAGAATGA GATGACAACA CCTTCTCTAG ATTATTCCAT
2051 GCCTGCTCCT TACAGGAGGG TAGAAGCACC TGTTGCCTAC CCAGAAGGGG
2101 AGAACAGCCA TGATAAATCG AGTTCTGAGA GAAGTACACC ACCATACCTT
2151 TTCCCAGAAT ACCCAGAAGC AAGCAAGAAT ACAGGTCAGA ATAGGGAGGT
2201 TTCAATTCTG TATCCAGGGG CCAAAGACCA ACGCCAGGGG TCCCTGCTTC
2251 CTGAAGAATT AGAAGATCAG ATGCCAAGAT TGGTGGCAGA AGAATCTAAC
2301 AGAGGTAGCA CAACCATAAA CAAAGAAGAA GTCAACAAGG GACCTTTTGT
2351 AGCTGTTGTG GGTGTTGCCA AAGGTGTTAG AGATTCAGGA GCTCCCATTC
2401 AGCTGATCCC TTTTAACAGA GAGGAGCTTG CTGAGAGACG AAAAGCAGTT
2451 GAATCCTGGA ACCCAGTGCC TTATTCTGTG GCCTCTGCTG CAATCCCTGC
2501 TGCAGCCATT GGGGAGAAAG CAAGAGGCTA TGAGGAGAGC GAAGGTCATA
2551 ATACACCAAA GTTGAAGAAC CAGAGAGAGC TGGAAGAATT GAAGAGAACC
2601 ACAGAAAAAT TGGAACGTGT TTTGGCTGAA AGGAATTTGT TCCAGCAAAA
2651 GGTGGAGGAG CTGGAACAGG AGAGGAATCA CTGGCAGTCT GAATTCAAGA
2701 AAGTCCAACA TGAATTGGTG ATCTACAGTA CCCAGGAGGC GGAAGGCTTG
2751 TACTGGAGCA AGAAACACAT GGGTTATCGC CAAGCTGAAT TCCAGATTCT
2801 GAAAGCTGAG CTGGAAAGAA CCAAAGAGGA AAAGCAAGAG TTAAAAGAGA
2851 AACTGAAGGA AACAGAGACA CACCTGGAAA TGCTGCAGAA GGCTCAGGTC
2901 TCCTACCGGA CCCCAGAGGG AGATGACCTA GAAAGGGCTT TGGCAAAGCT
2951 TACGCGGCTA CGTATCCACG TCAGCTATCT CCTTACTTCT GTCCTCCCTC
3001 ACTTGGAGCT TCGTGAGATC GGGTATGACT CAGAACAAGT GGATGGGATC
3051 CTGTACACGG TGCTGGAGGC AAATCACATA CTGGATTGAG CACCAGACTG
3101 TATACCCTTC TCTTCTCTTA TCTTCTGTCT GTTCTCTTTT CTCTCCCTCC
3151 CTCACGTCTC TCTCTCTCTC TCTCTCTCTC TCTCTCTCTC TCTCTCACCC
3201 TCACCTTTAT GCCTTATATA GAGAATCTCT GTGTAAATCC TGGCTCATAA
3251 TCAGTCTCCT TTTTATCAGT TTTGGTGTGG AGAAAGAGGC CAGTTTAAAT
3301 AGGCTTTCAA GAGTCTAGGG TCAGAAAAGC AATAGTCACT AAGCTAGGTG
3351 ACCTGAAAGC TTTAATTTTC ATGACCTGGA TATGTGGTCT ATTGTATATC
3401 TTTTTCTGAA ATGGTTTGTA TTCATTTAGG TTAGATCAAT CAGCAGATAT
3451 TGGGTCCGGT ATACCAGGTA TTATTTTGGG GTAAGCTAAC AAGTACAACT
3501 CATGTTTGCA GCCTTCGAAG ATGTAACAAT CTCGTTGGAA ACATAAGACA
3551 TACATCACAT TATACACAAA AGTGTATGAT ATGTACAACT GAGTGGTACA
3601 GATATGACAT GGAAGATCTG GGGGAGGAAG TTCAAGGAAG GCACCACACC
3651 AGAAATGGGA CCTAAATTAA GGCTTAAAGA ATGAGCATGG CCACACTGTC
3701 AGTGAGTGTT CTTTAGGTGG TAGGACTATG GTTGATATTT TTCTTCTTCC
3751 TATCTTCCTG CCTTTTTCAG ATTTTCAATA TTAAACTTGT TATTCTTACA
3801 TTGGAAAAAA ATAAATTGTT TTTAAAAAGA TC

## Fig. 7: SEQ ID NO. 3

**Length: 259 bp**

```
  1   GGAGAACCAG AGAGAGCTGG AAGAATTGAA GAGAACCACA GAAAAATTGG
 51   AACGTGTTTT GGCTGAAAGG AATTTGTTCC AGCAAAAGGT GGAGGAGCTG
101   GAACAGGAGA GGAATCACTG GCAGTCTGAA TTCAAGAAAG TCCAACATGA
151   ATTGGTGATC TACAGTACCC AGGAGGCGGA AGGCTTGTAC TGGAGCAAGA
201   AACACATGGG TTATCGCCAA GCTGAATTCC AGATTCTGAA AGCTGAGCTG
251   GAAAGAACC
```

# Fig. 8: Alignment of SEQ ID NO. 3 with human cDNA clone dJ75H8.2

**Length: 259 bp**

```
              .         .         .         .         .
   1 GGAGAACCAGAGAGAGCTGGAAGAATTGAAGAGAACCACAGAAAAATTGG 50
     | ||||||||||||||||||||||||||||||||||||||||||||||||
2564 GAAGAACCAGAGAGAGCTGGAAGAATTGAAGAGAACCACAGAAAAATTGG 2613
              .         .         .         .         .
  51 AACGTGTTTTGGCTGAAAGGAATTTGTTCCAGCAAAAGGTGGAGGAGCTG 100
     ||||||||||||||||||||||||||||||||||||||||||||||||||
2614 AACGTGTTTTGGCTGAAAGGAATTTGTTCCAGCAAAAGGTGGAGGAGCTG 2663
              .         .         .         .         .
 101 GAACAGGAGAGGAATCACTGGCAGTCTGAATTCAAGAAAGTCCAACATGA 150
     ||||||||||||||||||||||||||||||||||||||||||||||||||
2664 GAACAGGAGAGGAATCACTGGCAGTCTGAATTCAAGAAAGTCCAACATGA 2713
              .         .         .         .         .
 151 ATTGGTGATCTACAGTACCCAGGAGGCGGAAGGCTTGTACTGGAGCAAGA 200
     ||||||||||||||||||||||||||||||||||||||||||||||||||
2714 ATTGGTGATCTACAGTACCCAGGAGGCGGAAGGCTTGTACTGGAGCAAGA 2763
              .         .         .         .         .
 201 AACACATGGGTTATCGCCAAGCTGAATTCCAGATTCTGAAAGCTGAGCTG 250
     ||||||||||||||||||||||||||||||||||||||||||||||||||
2764 AACACATGGGTTATCGCCAAGCTGAATTCCAGATTCTGAAAGCTGAGCTG 2813

 251 GAAAGAACC 259
     |||||||||
2814 GAAAGAACC 2822
```

**Fig. 9: Schematic alignment of SEQ ID NO. 3 with Ensadin-0477 cDNA**

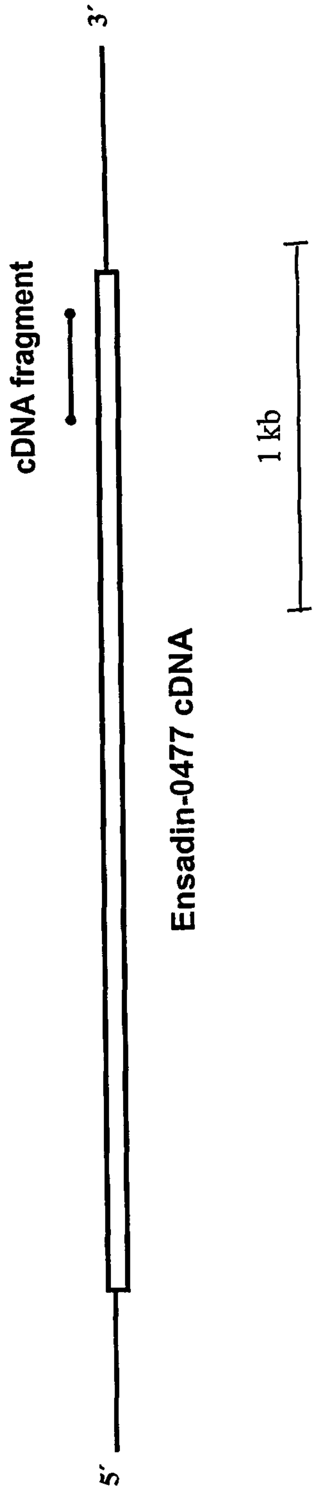

**Table 1 :**

| sample | | Δ (fold) (temporal / frontal cortex) |
|---|---|---|
| control | C011 | 0.78 |
| control | C012 | 0.78 |
| control | C014 | 1.03 |
| control | C005 | 0.86 |
| control | C008 | 1.27 |
| patient | P012 | 0.85 |
| patient | P016 | 1.91 |
| patient | P010 | 1.14 |
| patient | P011 | 1.85 |
| patient | P014 | 1.78 |
| patient | P017 | 1.52 |
| patient | P019 | 4.60 |

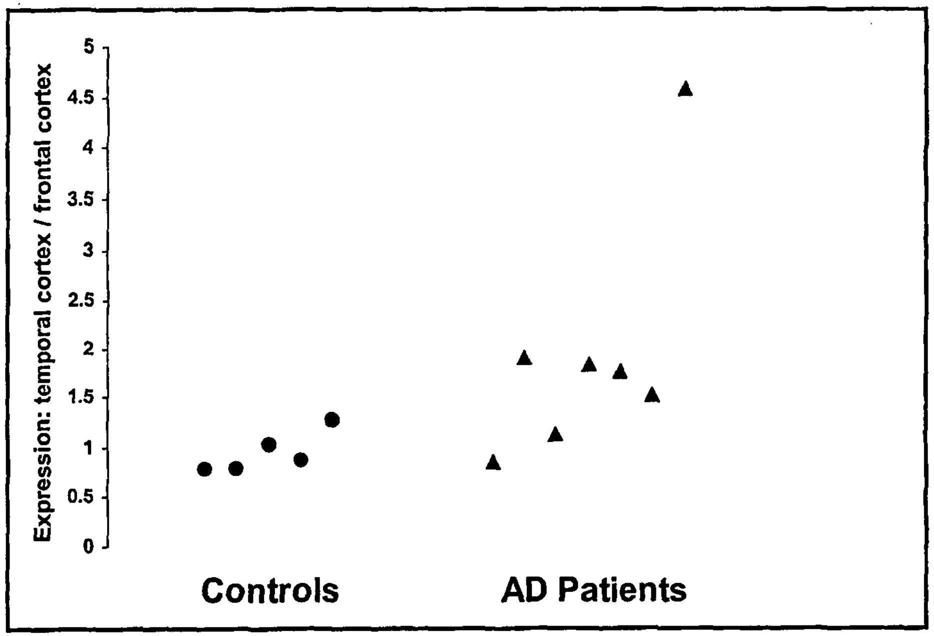

**Table 2:**

| sample | Δ (fold) (hippocampus / frontal cortex) |
|---|---|
| control C005 | 0.86 |
| control C008 | 1.74 |
| control C004 | 1.68 |
| patient P012 | 1.36 |
| patient P016 | 2.41 |
| patient P010 | 1.44 |
| patient P011 | 1.47 |
| patient P014 | 1.29 |
| patient P019 | 2.49 |

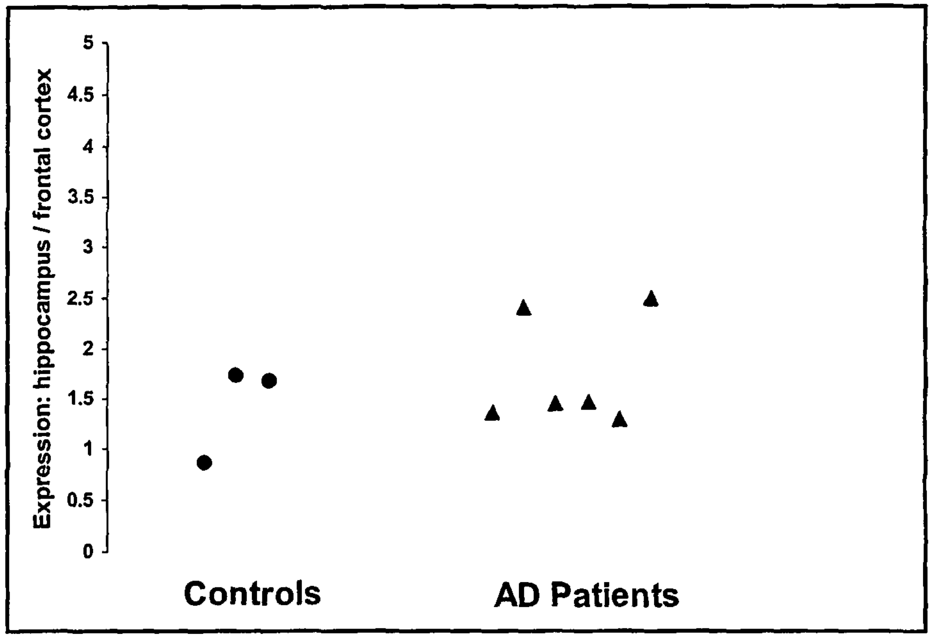

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0214543 A
- US 6150173 A
- WO 02009139 A


### Non-patent literature cited in the description


- **Vickers et al.** *Progress in Neurobiology,* 2000, vol. 60, 139-165
- **Selkoe.** *Physiological Rev,* 2001, vol. 81, 741-66
- **Greenfield et al.** *Frontiers Bioscience,* 2000, vol. 5, D72-83
- **Braak ; Braak.** *Acta Neuropathol,* 1991, vol. 82, 239-259
- **Johnson ; Jenkins.** *J Alzheimers Dis,* 1996, vol. 1, 38-58
- **Johnson ; Hartigan.** *J Alzheimers Dis,* 1999, vol. 1, 329-351
- **Schmitt et al.** *Neurology,* 2000, vol. 55, 370-376
- **Terry et al.** Annals of Neurology. 1981, vol. 10, 184-92
- **Strittmatter et al.** *Proc Natl Acad Sci USA,* 1993, vol. 90, 1977-81
- **Roses.** *Ann NY Acad Sci,* 1998, vol. 855, 738-43
- **Myers et al.** *Science,* 2000, vol. 290, 2304-5
- **Bertram et al.** *Science,* 2000, vol. 290, 2303
- **Scott et al.** *Am J Hum Genet,* 2000, vol. 66, 922-32
- **Iqbal ; Swaab ; Winblad ; Wisniewski.** Alzheimer's Disease and Related Disorders (Etiology, Pathogenesis and Therapeutics). Wiley & Sons, 1999
- **Scinto ; Daffner.** Early Diagnosis of Alzheimer's Disease. Humana Press, 2000
- **Mayeux ; Christen.** Epidemiology of Alzheimer's Disease: From Gene to Prevention. Springer Press, 1999
- **Younkin ; Tanzi ; Christen.** Presenilins and Alzheimer's Disease. Springer Press, 1998
- **Sambrook ; Russell.** Molecular Cloning: A Laboratory Manual. Cold Spring Harbor Laboratory Press, 2001
- **Schena M.** Microarray Biochip Technology. Eaton Publishing, 2000
- **Harlow ; Lane.** Using Antibodies: A Laboratory Manual. Cold Spring Harbor Laboratory Press, 1999
- **Edwards R.** Immunodiagnostics: A Practical Approach. Oxford University Press, 1999
- **Capecchi.** *Science,* 1989, vol. 244, 1288-1292
- **Hogan et al.** Manipulating the Mouse Embryo: A Laboratory Manual. Cold Spring Harbor Laboratory Press, 1994
- **Jackson ; Abbott.** Mouse Genetics and Transgenics: A Practical Approach. Oxford University Press, 1999
- **Harlow et al.** Antibodies, A Laboratory Manual. Cold Spring Harbor Laboratory Press, 1988
- **Dubel ; Breitling.** Recombinant Antibodies. Wiley-Liss, 1999
- **Edwards R.** Immunodiagnostics: A Practical Approach. Oxford University Press, 1999
- **Terry et al.** Annals of Neurology. 1981, vol. 10, 184-192
- **Strange.** Brain Biochemistry and Brain Disorders. Oxford University Press, 1992, 4
- **Liang ; Pardee.** *Science,* 1995, vol. 267, 1186-7
- **Kammer et al.** *Nucleic Acids Research,* 1999, vol. 27, 2211-2218
- **Liang et al.** *Nucleic Acids Research,* 1994, vol. 22, 5763-5764
- **Zhao et al.** *Biotechniques,* 1995, vol. 18, 842-850

SEQUENCE LISTING

<110> Evotec NeuroSciences GmbH

<120> Diagnostic and therapeutic use of Ensadin-0477 gene and protein for neurodegenerative diseases

<130> 021152ep ME/BM

<140> 02009139.3
<141> 2002-04-24

<160> 15

<170> PatentIn Ver. 2.1

<210> 1
<211> 937
<212> PRT
<213> Homo sapiens

<400> 1

Met Leu Leu Tyr Arg Gly Ala Pro Ala Gly Pro Gly Ala Pro Gly Cys
1 5 10 15

Gly Leu Ala Arg Pro Gly Gly Gly Pro Gln Ala Phe Gly Ile Arg Leu
20 25 30

Ser Thr Met Ser Pro Arg Tyr Leu Gln Ser Asn Ser Ser Ser His Thr
35 40 45

Arg Pro Phe Ser Ala Ile Ala Glu Leu Leu Asp Asn Ala Val Asp Pro
50 55 60

Asp Val Ser Ala Arg Thr Val Phe Ile Asp Val Glu Glu Val Lys Asn
65 70 75 80

Lys Ser Cys Leu Thr Phe Thr Asp Asp Gly Cys Gly Met Thr Pro His
85 90 95

Lys Leu His Arg Met Leu Ser Phe Gly Phe Thr Asp Lys Val Ile Lys
100 105 110

Lys Ser Gln Cys Pro Ile Gly Val Phe Gly Asn Gly Phe Lys Ser Gly
115 120 125

Ser Met Arg Leu Gly Lys Asp Ala Leu Val Phe Thr Lys Asn Gly Gly
130 135 140

Thr Leu Thr Val Gly Leu Leu Ser Gln Thr Tyr Leu Glu Cys Val Gln
145 150 155 160

Ala Gln Ala Val Ile Val Pro Ile Val Pro Phe Asn Gln Gln Asn Lys
165 170 175

Lys Met Ile Ile Thr Glu Asp Ser Leu Pro Ser Leu Glu Ala Ile Leu
180 185 190

Asn Tyr Ser Ile Phe Asn Arg Glu Asn Asp Leu Leu Ala Gln Phe Asp

195 200 205

Ala Ile Pro Gly Lys Lys Gly Thr Arg Val Leu Ile Trp Asn Ile Arg
210 215 220

Arg Asn Lys Asn Gly Lys Ser Glu Leu Asp Phe Asp Thr Asp Gln Tyr
225 230 235 240

Asp Ile Leu Val Ser Asp Phe Asp Thr Glu Glu Lys Met Thr Gly Gly
245 250 255

Val Thr Ser Glu Leu Pro Glu Thr Glu Tyr Ser Leu Arg Ala Phe Cys
260 265 270

Gly Ile Leu Tyr Met Lys Pro Arg Met Lys Ile Phe Leu Arg Gln Lys
275 280 285

Lys Val Thr Thr Gln Met Ile Ala Lys Ser Leu Ala Asn Val Glu Tyr
290 295 300

Asp Thr Tyr Lys Pro Thr Phe Thr Asn Lys Gln Val Arg Ile Thr Phe
305 310 315 320

Gly Phe Ser Cys Lys Asn Ser Asn Gln Phe Gly Ile Met Met Tyr His
325 330 335

Asn Asn Arg Leu Ile Lys Ser Phe Glu Lys Val Gly Cys Gln Val Lys
340 345 350

Pro Thr Arg Gly Glu Gly Val Gly Val Ile Gly Val Ile Glu Cys Asn
355 360 365

Phe Leu Lys Pro Ala Tyr Asn Lys Gln Asp Phe Glu Tyr Thr Lys Glu
370 375 380

Tyr Arg Leu Thr Ile Asn Ala Leu Ala Gln Lys Leu Asn Ala Tyr Trp
385 390 395 400

Lys Glu Lys Thr Ser Gln Asp Asn Phe Glu Thr Ser Thr Val Ala Arg
405 410 415

Pro Ile Pro Lys Val Pro Asp Gln Thr Trp Val Gln Cys Asp Glu Cys
420 425 430

Leu Lys Trp Arg Lys Leu Pro Gly Lys Ile Asp Pro Ser Met Leu Pro
435 440 445

Ala Arg Trp Phe Cys Tyr Tyr Asn Ser His Pro Lys Tyr Arg Arg Cys
450 455 460

Ser Val Pro Glu Glu Gln Glu Leu Thr Asp Glu Asp Leu Cys Leu Ser
465 470 475 480

Lys Ala Lys Lys Gln Glu Gln Thr Val Glu Glu Lys Lys Lys Met Pro
485 490 495

Met Glu Asn Glu Asn His Gln Val Phe Ser Asn Pro Pro Lys Ile Leu
500 505 510

```
Thr Val Gln Glu Met Ala Gly Leu Asn Asn Lys Thr Ile Gly Tyr Glu
        515                 520                 525

Gly Ile His Ser Pro Ser Val Leu Pro Ser Gly Gly Glu Glu Ser Arg
    530                 535                 540

Ser Pro Ser Leu Gln Leu Lys Pro Leu Asp Ser Ser Val Leu Gln Phe
545                 550                 555                 560

Ser Ser Lys Tyr Lys Trp Ile Leu Gly Glu Glu Pro Val Glu Lys Arg
                565                 570                 575

Arg Arg Leu Gln Asn Glu Met Thr Thr Pro Ser Leu Asp Tyr Ser Met
            580                 585                 590

Pro Ala Pro Tyr Arg Arg Val Glu Ala Pro Val Ala Tyr Pro Glu Gly
        595                 600                 605

Glu Asn Ser His Asp Lys Ser Ser Ser Glu Arg Ser Thr Pro Pro Tyr
    610                 615                 620

Leu Phe Pro Glu Tyr Pro Glu Ala Ser Lys Asn Thr Gly Gln Asn Arg
625                 630                 635                 640

Glu Val Ser Ile Leu Tyr Pro Gly Ala Lys Asp Gln Arg Gln Gly Ser
                645                 650                 655

Leu Leu Pro Glu Glu Leu Glu Asp Gln Met Pro Arg Leu Val Ala Glu
            660                 665                 670

Glu Ser Asn Arg Gly Ser Thr Thr Ile Asn Lys Glu Glu Val Asn Lys
        675                 680                 685

Gly Pro Phe Val Ala Val Val Gly Val Ala Lys Gly Val Arg Asp Ser
    690                 695                 700

Gly Ala Pro Ile Gln Leu Ile Pro Phe Asn Arg Glu Glu Leu Ala Glu
705                 710                 715                 720

Arg Arg Lys Ala Val Glu Ser Trp Asn Pro Val Pro Tyr Ser Val Ala
                725                 730                 735

Ser Ala Ala Ile Pro Ala Ala Ala Ile Gly Glu Lys Ala Arg Gly Tyr
            740                 745                 750

Glu Glu Ser Glu Gly His Asn Thr Pro Lys Leu Lys Asn Gln Arg Glu
        755                 760                 765

Leu Glu Glu Leu Lys Arg Thr Thr Glu Lys Leu Glu Arg Val Leu Ala
    770                 775                 780

Glu Arg Asn Leu Phe Gln Gln Lys Val Glu Glu Leu Glu Gln Glu Arg
785                 790                 795                 800

Asn His Trp Gln Ser Glu Phe Lys Lys Val Gln His Glu Leu Val Ile
                805                 810                 815

Tyr Ser Thr Gln Glu Ala Glu Gly Leu Tyr Trp Ser Lys Lys His Met
            820                 825                 830
```

```
Gly Tyr Arg Gln Ala Glu Phe Gln Ile Leu Lys Ala Glu Leu Glu Arg
        835                 840                     845

Thr Lys Glu Glu Lys Gln Glu Leu Lys Glu Lys Leu Lys Glu Thr Glu
    850                 855                     860

Thr His Leu Glu Met Leu Gln Lys Ala Gln Val Ser Tyr Arg Thr Pro
865                 870                 875                 880

Glu Gly Asp Asp Leu Glu Arg Ala Leu Ala Lys Leu Thr Arg Leu Arg
                885                 890                 895

Ile His Val Ser Tyr Leu Leu Thr Ser Val Leu Pro His Leu Glu Leu
            900                 905                 910

Arg Glu Ile Gly Tyr Asp Ser Glu Gln Val Asp Gly Ile Leu Tyr Thr
        915                 920                 925

Val Leu Glu Ala Asn His Ile Leu Asp
    930                 935
```

<210> 2
<211> 3832
<212> DNA
<213> Artificial Sequence

<220>
<223> Description of Artificial Sequence: cDNA of the human Ensadin-0477 gene

<400> 2

```
atatttctgt acggcccaga ggcagcccgg gcagtcgccg ggtcggtaga ggtgctgaca 60
gggcggagcc gccgcttgcc tcagccggcc tcacccctcc cgcccgggac aggtggctgg 120
agcgcgctcc gcctccgcca gctgcgagtg ggagcgaggg agggccggtc ccggacggtc 180
gccgccagct gcgagtggaa gtgagcgagg gctagtcccg gaccgccggg ccaggggtcc 240
ggcggcagca gacgggtacc gtggcggcca aaaaaatgct cctgtaccga ggggcccccg 300
ccggccctgg cgcgccgggc tgcgggctgg cccggcccgg cggcggcccg caggccttcg 360
ggatccgcct gagcacgatg agcccccgct acctccagag caactccagc agccacacgc 420
gacccttcag tgccatcgcg gagctgctag ataatgctgt agatccagat gtatctgcca 480
ggacggtctt tatagatgtt gaggaggtca agaataaatc ttgtttgacc tttaccgatg 540
atggatgtgg gatgacacct cataaactac accgaatgct cagctttggc tttacagata 600
aagtaataaa gaagagccag tgtcccattg gggtctttgg taatggtttc aagtcaggct 660
ccatgcggct aggaaaggac gcccttgtct tcaccaagaa tgggggtact ctcactgttg 720
gacttctatc acagacctat ctggaatgtg tccaggccca ggcagttatt gtaccaattg 780
ttccattcaa ccagcaaaac aaaaaaatga ttattaccga ggattcattg cccagcctag 840
aagccatctt gaactattcc attttcaacc gtgaaaatga cctgctggcc cagtttgatg 900
ccatcccagg caaaaaaggc actcgtgttc tcatttggaa catccgcaga aataaaaatg 960
gaaaatctga gttggacttt gatacagatc aatatgacat cctggtatca gactttgaca 1020
cagaagaaaa aatgactggc ggtgttacct ctgagctacc agaaacagaa tattctttaa 1080
gggcattttg tggtattcta tacatgaagc cacgcatgaa aatttttctg cgtcaaaaga 1140
aggtgactac ccagatgatt gccaagagcc tggccaatgt agaatatgat acatataaac 1200
ctaccttcac aaataagcag gtgagaatca cctttgggtt ctcttgcaag aatagtaacc 1260
agtttggaat aatgatgtat cataacaacc gactcataaa atcttttgag aaggtggggt 1320
gccaggtgaa gccaactcgt ggagaaggtg taggagtaat tggagtcatt gagtgcaatt 1380
tcctaaaacc tgcctacaac aaacaagact ttgagtatac caaggagtac cggctaacaa 1440
taaatgccct tgcccagaag ctcaatgctt actggaagga aaaaacatct caagataatt 1500
ttgagacctc aactgtagcc aggccaatac cgaaggttcc tgaccagaca tgggttcagt 1560
```

```
gtgatgagtg tcttaaatgg agaaagcttc ctgggaagat tgatccatcc atgttacctg 1620
caagatggtt ttgttattat aattcccatc caaagtacag gagatgctct gttccagagg 1680
aacaagaact cactgatgaa gacctgtgct tgagcaaagc taagaaacaa gaacaaactg 1740
ttgaggagaa gaagaagatg cctatggaaa atgagaacca ccaggtattc agtaatccac 1800
caaagatcct tactgttcaa gaaatggctg gattgaataa caagacaatt ggatatgagg 1860
gaattcatag ccctagtgtg cttccttctg gtggagaaga aagcagatca ccatctcttc 1920
aacttaagcc tctggattcc agtgttttac agttttccag taagtacaaa tggatcctag 1980
gtgaagaacc ggtggagaaa cgaagaaggc tccagaatga gatgacaaca ccttctctag 2040
attattccat gcctgctcct tacaggaggg tagaagcacc tgttgcctac ccagaagggg 2100
agaacagcca tgataaatcg agttctgaga gaagtacacc accatacctt ttcccagaat 2160
acccagaagc aagcaagaat acaggtcaga atagggaggt ttcaattctg tatccagggg 2220
ccaaagacca acgccagggg tccctgcttc ctgaagaatt agaagatcag atgccaagat 2280
tggtggcaga agaatctaac agaggtagca caaccataaa caaagaagaa gtcaacaagg 2340
gaccttttgt agctgttgtg ggtgttgcca aaggtgttag agattcagga gctcccattc 2400
agctgatccc ttttaacaga gaggagcttg ctgagagacg aaaagcagtt gaatcctgga 2460
acccagtgcc ttattctgtg gcctctgctg caatccctgc tgcagccatt ggggagaaag 2520
caagaggcta tgaggagagc gaaggtcata atacaccaaa gttgaagaac cagagagagc 2580
tggaagaatt gaagagaacc acagaaaaat tggaacgtgt tttggctgaa aggaatttgt 2640
tccagcaaaa ggtggaggag ctggaacagg agaggaatca ctggcagtct gaattcaaga 2700
aagtccaaca tgaattggtg atctacagta cccaggaggc ggaaggcttg tactggagca 2760
agaaacacat gggttatcgc caagctgaat tccagattct gaaagctgag ctggaaagaa 2820
ccaaagagga aaagcaagag ttaaaagaga aactgaagga aacagagaca cacctggaaa 2880
tgctgcagaa ggctcaggtc tcctaccgga ccccagaggg agatgaccta gaaagggctt 2940
tggcaaagct tacgcggcta cgtatccacg tcagctatct ccttacttct gtcctccctc 3000
acttggagct tcgtgagatc gggtatgact cagaacaagt ggatgggatc ctgtacacgg 3060
tgctggaggc aaatcacata ctggattgag caccagactg tataccccttc tcttctctta 3120
tcttctgtct gttctctttt ctctccctcc ctcacgtctc tctctctctc tctctctctc 3180
tctctctctc tctctcaccc tcacctttat gccttatata gagaatctct gtgtaaatcc 3240
tggctcataa tcagtctcct ttttatcagt tttggtgtgg agaaagaggc cagtttaaat 3300
aggctttcaa gagtctaggg tcagaaaagc aatagtcact aagctaggtg acctgaaagc 3360
tttaattttc atgacctgga tatgtggtct attgtatatc tttttctgaa atggtttgta 3420
ttcatttagg ttagatcaat cagcagatat tgggtccggt ataccaggta ttattttggg 3480
gtaagctaac aagtacaact catgtttgca gccttcgaag atgtaacaat ctcgttggaa 3540
acataagaca tacatcacat tatacacaaa agtgtatgat atgtacaact gagtggtaca 3600
gatatgacat ggaagatctg gggaggaaag ttcaaggaag gcaccacacc agaaatggga 3660
cctaaattaa ggcttaaaga atgagcatgg ccacactgtc agtgagtgtt ctttaggtgg 3720
taggactatg gttgatattt ttcttcttcc tatcttcctg ccttttttcag attttcaata 3780
ttaaacttgt tattcttaca ttggaaaaaa ataaattgtt tttaaaaaga tc         3832
```

<210> 3
<211> 259
<212> DNA
<213> Artificial Sequence

<220>
<223> Description of Artificial Sequence: cDNA fragment of the human Ensadin-0477 gene

<400> 3

```
ggagaaccag agagagctgg aagaattgaa gagaaccaca gaaaaattgg aacgtgtttt 60
ggctgaaagg aatttgttcc agcaaaaggt ggaggagctg gaacaggaga ggaatcactg 120
gcagtctgaa ttcaagaaag tccaacatga attggtgatc tacagtaccc aggaggcgga 180
aggcttgtac tggagcaaga aacacatggg ttatcgccaa gctgaattcc agattctgaa 240
agctgagctg gaaagaacc                                              259
```

<210> 4
<211> 20
<212> DNA
<213> Artificial Sequence

<220>
<223> Description of Artificial Sequence: Primer for the human Ensadin-0477 gene

<400> 4
ccacaccaga aatgggacct 20

<210> 5
<211> 22
<212> DNA
<213> Artificial Sequence

<220>
<223> Description of Artificial Sequence: Primer for the human Ensadin-0477 gene

<400> 5
aacactcact gacagtgtgg cc 22

<210> 6
<211> 20
<212> DNA
<213> Artificial Sequence

<220>
<223> Description of Artificial Sequence: Primer for the cyclophilin B gene

<400> 6
actgaagcac tacgggcctg 20

<210> 7
<211> 19
<212> DNA
<213> Artificial Sequence

<220>
<223> Description of Artificial Sequence: Primer for the cyclophilin B gene

<400> 7
agccgttggt gtctttgcc 19

<210> 8
<211> 20
<212> DNA
<213> Artificial Sequence

<220>

<223> Description of Artificial Sequence: Primer for the ribosomal protein S9

<400> 8
ggtcaaattt accctggcca 20

<210> 9
<211> 22
<212> DNA
<213> Artificial Sequence

<220>
<223> Description of Artificial Sequence: Primer for the ribosomal protein S9

<400> 9
tctcatcaag cgtcagcagt tc 22

<210> 10
<211> 19
<212> DNA
<213> Artificial Sequence

<220>
<223> Description of Artificial Sequence: Primer for the beta-actin gene

<400> 10
tggaacggtg aaggtgaca 19

<210> 11
<211> 19
<212> DNA
<213> Artificial Sequence

<220>
<223> Description of Artificial Sequence: Primer for the beta-actin gene

<400> 11
ggcaagggac ttcctgtaa 19

<210> 12
<211> 20
<212> DNA
<213> Artificial Sequence

<220>
<223> Description of Artificial Sequence: Primer for the GAPDH gene

<400> 12
cgtcatgggt gtgaaccatg 20

<210> 13
<211> 21
<212> DNA
<213> Artificial Sequence

<220>
<223> Description of Artificial Sequence: Primer for the GAPDH gene

<400> 13

gctaagcagt tggtggtgca g 21

<210> 14
<211> 21
<212> DNA
<213> Artificial Sequence

<220>
<223> Description of Artificial Sequence: Primer for the transferrin receptor TRR

<400> 14
gtcgctggtc agttcgtgat t 21

<210> 15
<211> 23
<212> DNA
<213> Artificial Sequence

<220>
<223> Description of Artificial Sequence: Primer for the transferrin receptor TRR

<400> 15
agcagttggc tgttgtacct ctc 23